# EUROPÄISCHE PATENTSCHRIFT

(11) **EP 1 628 962 B1**
(45) Veröffentlichungstag und Bekanntmachung des Hinweises auf die Patenterteilung: **01.10.2014**
(21) Anmeldenummer: 04732311.8
(22) Anmeldetag: 12.05.2004
(51) Int. Cl.: C07D 231/16

(54) **Iodpyrazolylcarboxanilide**
Iodopyrazolyl carboxanilides
Carboxanilides de iodopyrazole

(30) Priorität: 21.05.2003 DE 10322910; 05.06.2003 DE 10325439
(43) Veröffentlichungstag der Anmeldung: 01.03.2006
(73) Patentinhaber: Bayer Intellectual Property GmbH, 40789 Monheim (DE)
(72) Erfinder: DUNKEL, Ralf, 69001 Lyon (FR); ELBE, Hans-Ludwig, 42329 Wuppertal (DE); GREUL, Jörg, Nico, 42799 Leichlingen (DE); HARTMANN, Benoit, 40764 Langenfeld (DE); WACHENDORFF-NEUMANN, Ulrike, 56566 Neuwied (DE); DAHMEN, Peter, 41470 Neuss (DE); KUCK, Karl-Heinz, 40764 Langenfeld (DE)
(74) Vertreter: BIP Patents
(86) Internationale Anmeldenummer: PCT/EP2004/005066
(87) Internationale Veröffentlichungsnummer: WO 2004/103975

(56) Entgegenhaltungen:
- EP-A1- 0 199 822
- WO-A-93/11117
- DE-A- 10 136 065
- J. A. ERICKSON ET AL.: "Hydrogen Bond Donor Properties of the Difluoromethyl Group" JOURNAL OF ORGANIC CHEMISTRY., Bd. 60, Nr. 6, 1995, Seiten 1626-1631, XP002296451 USAMERICAN CHEMICAL SOCIETY, WASHINGTON, DC.

## Beschreibung

Die vorliegende Erfindung betrifft neue Iodpyrazolylcarboxanilide, mehrere Verfahren zu deren Herstellung und deren Verwendung zur Bekämpfung von unerwünschten Mikroorganismen.

Es ist bereits bekannt, dass zahlreiche Carboxanilide fungizide Eigenschaften besitzen (vgl. z.B. WO 93/11117, EP-A 0 589 301, EP-A 0 545 099, JP-A 2001-302605, JP-A 10-251240 und JP-A 8-176112). So sind aus WO 93/11117 bereits *N*-(2-Cyclopentylphenyl)-3-iod-1-methyl-1*H*-pyrazol-4-carboxamid, *N*-(2-Cyclohexylphenyl)-3-iod-1-methyl-1*H*-pyrazol-4-carboxamid, *N*-(2-Cycloheptyl-phenyl)-3-iod-1-methyl-1*H*-pyrazol-4-carboxamid, *N*-(2-Cyclooctylphenyl)-3-iod-1-methyl-1*H*-pyrazol-4-carboxamid und *N*-(2-Bicyclo[2.2.1]hept-2-ylphenyl)-3-iod-1-methyl-1*H*-pyrazol-4-carboxamid, aus EP-A 0 589 301 *N*-(4'-Chlor-1,1'-biphenyl-2-yl)-1,3-dimethyl-1*H*-pyrazol-4-carboxamid und aus JP-A 10-251240 *N*-[2-(1,3-Dimethylbutyl)phenyl]-1,3-dimethyl-1*H*-pyrazol-4-carboxamid bekannt. Die Wirksamkeit dieser Stoffe ist gut, lässt aber in manchen Fällen, z.B. bei niedrigen Aufwandmengen zu wünschen übrig.

Es wurden nun neue Iodpyrazolylcarboxanilide der Formel (I) 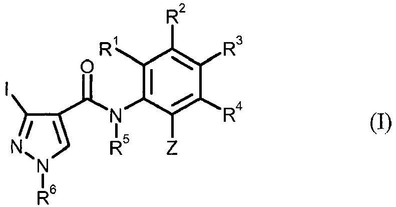 gefunden, in welcher
- R¹, R², R³ und R⁴: unabhängig voneinander für Wasserstoff, Fluor, Chlor, Methyl, iso-Propyl oder Methylthio stehen,
- R⁵: für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁷, -CONR⁸R⁹ oder -CH₂NR¹⁰R¹¹ steht,
- R⁶: für C₁-C₃-Alkyl, C₁-C₂-Alkoxy-C₁-C₂-alkyl, C₁-C₃-Halogenalkyl mit 1 bis 7 Fluor-, Chlor- und/oder Bromatomen steht,
- R⁷: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈- Halogencycloalkyl mit jeweils 1 bis 9 Fluor-, Chlor- und/oder Bromatomen, -COR¹² steht,
- R⁸ und R⁹: unabhängig voneinander für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencyclo-alkyl mit jeweils 1 bis 9 Fluor-, Chlor- und/oder Bromatomen stehen,
- R⁸ und R⁹: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹³ enthalten kann,
- R¹⁰ und R¹¹: unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
- R¹⁰ und R¹¹: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹³ enthalten kann,
- R¹²: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
- R¹³: für Wasserstoff oder C₁-C₆-Alkyl steht,
- Z: für Z¹ steht, worin
- Z¹: für gegebenenfalls einfach bis fünffach, gleich oder verschieden substituiertes Phenyl steht,
oder
- R¹, R² und R³: unabhängig voneinander für Wasserstoff, Fluor oder Chlor stehen und
- Z und R⁴: gemeinsam mit den Kohlenstoffatomen, an die sie gebunden sind, einen gegebenenfalls substituierten 5- oder 6-gliedrigen carbocyclischen oder heterocyclischen Ring bilden.

Weiterhin wurde gefunden, dass man Iodpyrazolylcarboxanilide der Formel (I) erhält, indem man
a) Iodpyrazolylcarbonsäurederivate der Formel (II) 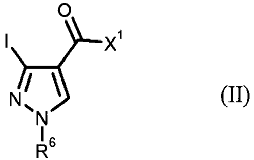 in welcher
   - R⁶: die oben angegebenen Bedeutungen hat und
   - X¹: für Chlor oder Hydroxy steht,
   mit Anilin-Derivaten der Formel (III) 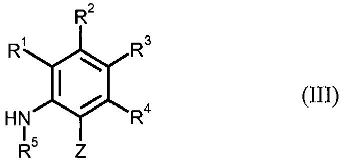 in welcher R¹, R², R³, R⁴, R⁵ und Z die oben angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt,
   oder
b) Halogen-iodpyrazolylcarboxanilide der Formel (IV) 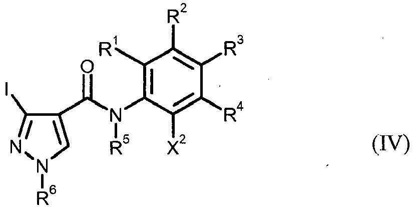 in welcher
   R¹, R², R³, R⁴, R⁵ und R⁶ die oben angegebenen Bedeutungen haben, und
   X² für Chlor, Brom, Iod oder Trifluormethylsulfonat steht,
   mit Boronsäure-Derivaten der Formel (V) 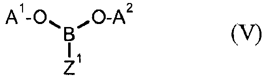 in welcher
   Z¹ die oben angegebene Bedeutung hat und
   A¹ und A² jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt,
   oder
c) Iodpyrazolylcarboxamid-Boronsäure-Derivate der Formel (VI) 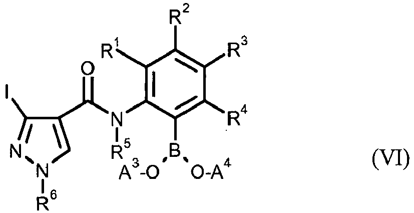 in welcher
   R¹, R², R³, R⁴, R⁵ und R⁶ die oben angegebenen Bedeutungen haben, und
   A³ und A⁴ jeweils für Wasserstoff oder zusammen für Tetramethylethylen stehen,
   mit Phenyl-Derivaten der Formel (VII)

   X³-Z¹ (VII)

   in welcher
   Z¹ die oben angegebenen Bedeutungen hat und
   X³ für Chlor, Brom, Iod oder Trifluormethylsulfonat steht,
   in Gegenwart eines Katalysators, gegebenenfalls in Gegenwart eines Säurebindemittels und gegebenenfalls in Gegenwart eines Verdünnungsmittels umsetzt,
   oder
d) Halogen-iodpyrazolylcarboxanilide der Formel (IV) 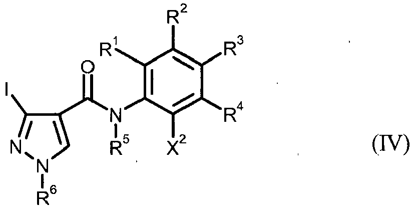 in welcher
   R¹, R², R³, R⁴, R⁵ und R⁶ die oben angegebenen Bedeutungen haben, und
   X² für Chlor, Brom, Iod oder Trifluormethylsulfonat steht,
   mit Phenyl-Derivaten der Formel (VII)

   X³-Z¹ (VII)

   in welcher
   Z¹ die oben angegebenen Bedeutungen hat und
   X³ für Chlor, Brom, Iod oder Trifluormethylsulfonat steht,
   in Gegenwart eines Palladium- oder Nickel-Katalysators und in Gegenwart von 4,4,4',4',5,5,5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt,
   oder
e) Iodpyrazolylcarboxanilide der Formel (Ia) 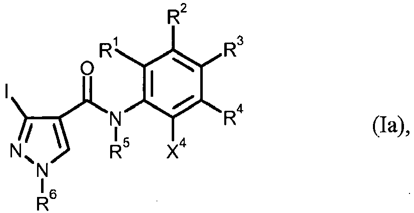 in welcher
   - R¹, R², R³, R⁴, R⁵ und R⁶: die oben angegebenen Bedeutungen haben und
   - X⁴: für jeweils gegebenenfalls einfach oder mehrfach, gleich oder verschieden durch Halogen und/oder C₃-C₆-Cycloalkyl substituiertes C₂-C₂₀-Alkenyl oder C₂-C₂₀-Alkinyl steht, wobei der Cycloalkylteil seinerseits gegebenenfalls durch Halogen und/oder C₁-C₄-Alkyl substituiert sein kann,
   gegebenenfalls in Gegenwart eines Verdünnungsmittels und gegebenenfalls in Gegenwart eines Katalysators hydriert,
   oder
f) Hydroxyalkyl-iodpyrazolylcarboxanilide der Formel (VIII) 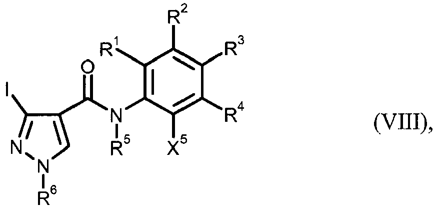 in welcher
   - R¹, R², R³, R⁴, R⁵ und R⁶: die oben angegebenen Bedeutungen haben und
   - X⁵: für gegebenenfalls zusätzlich einfach oder mehrfach, gleich oder verschieden durch Halogen und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann,
   gegebenenfalls in Gegenwart eines Verdünnungsmittels und gegebenenfalls in Gegenwart einer Säure dehydratisiert,
   oder
g) Halogen-iodpyrazolylcarboxanilide der Formel (IV) 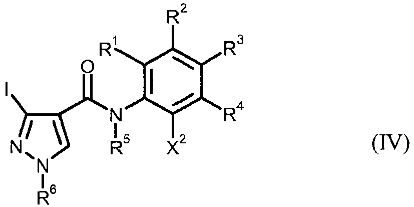 in welcher
   R¹, R², R³, R⁴, R⁵ und R⁶ die oben angegebenen Bedeutungen haben, und
   X² für Chlor, Brom, Iod oder Trifluormethylsulfonat steht,
   mit einem Alkin der Formel (IX) 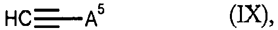 in welcher
   - A⁵: für jeweils gegebenenfalls einfach oder mehrfach, gleich oder verschieden durch Halogen und/oder C₃-C₆-Cycloalkyl substituiertes C₂-C₁₈-Alkyl steht, wobei der Cycloalkylteil seinerseits gegebenenfalls durch Halogen und/oder C₁-C₄-Alkyl substituiert sein kann,
   oder einem Alken der Formel (X) 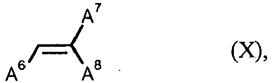 in welcher
   - A⁶, A⁷ und A⁸: unabhängig voneinander jeweils für Wasserstoff oder gegebenenfalls einfach oder mehrfach, gleich oder verschieden durch Halogen und/oder C₃-C₆-Cycloalkyl substituiertes Alkyl stehen, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils die Zahl 20 nicht übersteigt,
   gegebenenfalls in Gegenwart eines Verdünnungsmittels, gegebenenfalls in Gegenwart eines Säurebindemittels und in Gegenwart eines oder mehrerer Katalysatoren umsetzt,
   oder
h) Ketone der Formel (XI) 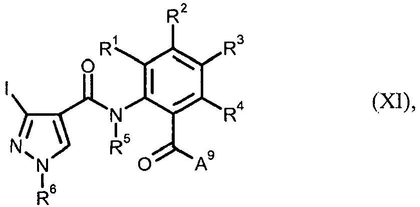 in welcher
   - R¹, R², R³, R⁴, R⁵ und R⁶: die oben angegebenen Bedeutungen haben und
   - A⁹: für Wasserstoff oder gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
   mit einer Phosphorverbindung der allgemeinen Formel (XII)

   A¹⁰-Px (XII),

   in welcher
   - A¹⁰: für gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
   - Px: für eine Gruppierung -P⁺(C₆H₅)₃ Cl⁻, -P⁺(C₆H₅)₃ Br⁻, -P⁺(C₆H₅)₃ I⁻, -P(=O)(OCH₃)₃ oder -P(=O)(OC₂H₅)₃ steht,
   gegebenenfalls in Gegenwart eines Verdünnungsmittels umsetzt,
   oder
i) Iodpyrazolylcarboxanilide der Formel (Ib) 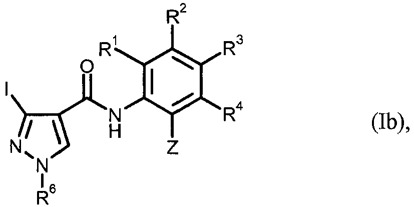 in welcher
   R¹, R², R³, R⁴, R⁶ und Z die oben angegebenen Bedeutungen haben
   mit einem Halogenid der Formel (XIII)

   R⁵⁻¹-X⁶ R⁵⁻¹-X⁶ (XIII)

   in welcher
   - R⁵⁻¹: für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/ oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁷, -CONR⁸R⁹ oder -CH₂NR¹⁰R¹¹ steht,
   - R⁷, R⁸, R⁹, R¹⁰ und R¹¹: die oben angegebenen Bedeutungen haben und
   - X⁶: für Chlor, Brom oder Iod steht,
   in Gegenwart einer Base und in Gegenwart eines Verdünnungsmittels umsetzt.

Schließlich wurde gefunden, dass die neuen Iodpyrazolylcarboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Iodpyrazolylcarboxanilide der Formel (I) eine wesentlich bessere fungizide Wirksamkeit als die konstitutionell ähnlichsten, vorbekannten Wirkstoffe gleicher Wirkungsrichtung.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Iodpyrazolylcarboxanilide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- R¹, R², R³ und R⁴: stehen unabhängig voneinander bevorzugt für Wasserstoff, Fluor, Chlor oder Methyl.
- R¹: steht besonders bevorzugt für Wasserstoff oder Fluor.
- R¹: steht ganz besonders bevorzugt für Wasserstoff.
- R¹: steht auch ganz besonders bevorzugt für Fluor.
- R²: steht besonders bevorzugt für Wasserstoff.
- R³: steht besonders bevorzugt für Wasserstoff, Fluor, Chlor oder Methylthio.
- R³: steht ganz besonders bevorzugt für Wasserstoff.
- R³: steht auch ganz besonders bevorzugt für Fluor.
- R⁴: steht besonders bevorzugt für Wasserstoff, Methyl oder iso-Propyl.
- R⁴: steht ganz besonders bevorzugt für Wasserstoff.
- R⁴: steht auch ganz besonders bevorzugt für Methyl.
- R¹, R², R³ und R⁴: stehen ganz besonders bevorzugt gleichzeitig für Wasserstoff.
- R⁵: steht bevorzugt für Wasserstoff; C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₆-Halogencycloalkyl mit jeweils 1 bis 9 Fluor-, Chlor- und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)-carbonyl-C₁-C₃-halogenalkyl mit jeweils 1 bis 13 Fluor-, Chlor- und/oder Bromatomen; -COR⁷, -CONR⁸R⁹ oder -CH₂NR¹⁰R¹¹.
- R⁵: steht besonders bevorzugt für Wasserstoff, Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl, -CH₂-CHO, -CH₂CH₂-CHO, -CH₂-CO-CH₃, -CH₂-CO-CH₂CH₃, -CH₂-CO-CH(CH₃)₂, -CH₂CH₂-CO-CH₃, -CH₂CH₂-CO-CH₂CH₃,-CH₂CH₂-CO-CH(CH₃)₂, -CH₂-C(O)OCH₃, -CH₂-C(O)OCH₂CH₃, -CH₂-C(O)OCH(CH₃)₂, -CH₂CH₂-C(O)OCH₃, -CH₂CH₂-C(O)OCH₂CH₃, -CH₂CH₂-C(O)OCH(CH ₃)₂, -CH₂-CO-CF₃, -CH₂-CO-CCl₃, -CH₂-CO-CH₂CF₃, -CH₂-CO-CH₂CCl₃, -CH₂CH₂-CO-CH₂CF₃, -CH₂CH₂-CO-CH₂CCl₃, -CH₂-C(O)OCH₂CF₃, -CH₂-C(O)OCF₂CF₃, -CH₂-C(O)OCH₂CCl₃, -CH₂-C(O)OCCl₂CCl₃, -CH₂CH₂-C(O)OCH₂CF₃, -CH₂CH₂-C(O)OCF₂CF₃, -CH₂CH₂-C(O)OCH₂CCl₃, -CH₂CH₂-C(O)O-CCl₂CCl₃; -COR⁷, -CONR⁸R⁹ oder -CH₂NR¹⁰R¹¹.
- R⁵: steht ganz besonders bevorzugt für Wasserstoff; Methyl, Methoxymethyl, -CH₂-CHO, -CH₂CH₂-CHO, -CH₂-CO-CH₃, -CH₂-CO-CH₂CH₃, -CH₂-CO-CH(CH₃)₂ oder -COR⁷.
- R⁶: steht bevorzugt für Methyl, Ethyl, iso-Propyl, Monofluormethyl, Difluormethyl oder Trifluormethyl.
- R⁶: steht besonders bevorzugt für Methyl, Ethyl oder iso-Propyl.
- R⁶: steht ganz besonders bevorzugt für Methyl.
- R⁷: steht bevorzugt für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen, -COR¹².
- R⁷: steht besonders bevorzugt für Wasserstoff, Methyl, Ethyl, n- oder iso-Propyl, tert-Butyl, Methoxy, Ethoxy, iso-Propoxy, tert-Butoxy, Cyclopropyl; Trifluormethyl, Trifluormethoxy, -COR¹².
- R⁷: steht ganz besonders bevorzugt für Wasserstoff, -COCH₃,-CHO, -COCH₂OCH₃, -COCO₂CH₃, -COCO₂CH₂CH₃.
- R⁸ und R⁹: stehen unabhängig voneinander bevorzugt für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R⁸ und R⁹: bilden außerdem gemeinsam mit dem Stickstoffatom, an das sie gebunden sind, bevorzugt einen gegebenenfalls einfach bis vierfach, gleich oder verschieden durch Halogen oder C₁-C₄-Alkyl substituierten gesättigten Heterocyclus mit 5 bis 8 Ringatomen, wobei der Heterocyclus 1 oder 2 weitere, nicht benachbarte Heteroatome aus der Reihe Sauerstoff, Schwefel oder NR¹³ enthalten kann.
- R⁸ und R⁹: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁸ und R⁹: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹³ substituiert sein kann.
- R¹⁰ und R¹¹: stehen unabhängig voneinander bevorzugt für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor- und/oder Bromatomen.
- R¹⁰ und R¹¹: bilden außerdem gemeinsam mit dem Stickstoffatom, an das sie gebunden sind, bevorzugt einen gegebenenfalls einfach oder mehrfach, gleich oder verschieden durch Halogen oder C₁-C₄-Alkyl substituierten gesättigten Heterocyclus mit 5 bis 8 Ringatomen, wobei der Heterocyclus 1 oder 2 weitere, nicht benachbarte Heteroatome aus der Reihe Sauerstoff, Schwefel oder NR¹³ enthalten kann.
- R¹⁰ und R¹¹: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R¹⁰ und R¹¹: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹³ substituiert sein kann.
- R¹²: steht bevorzugt für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen.
- R¹²: steht besonders bevorzugt für Wasserstoff, Methyl, Ethyl, n- oder iso-Propyl, tert-Butyl, Methoxy, Ethoxy, iso-Propoxy, tert-Butoxy, Cyclopropyl; Trifluormethyl, Trifluormethoxy.
- R¹³: steht bevorzugt für Wasserstoff oder C₁-C₄-Alkyl.
- R¹³: steht besonders bevorzugt für Wasserstoff, Methyl, Ethyl, n- oder iso-Propyl, n-, iso-, sec-oder tert-Butyl.
- Z: steht bevorzugt für Z¹.
- Z¹: steht bevorzugt für gegebenenfalls einfach bis fünffach, gleich oder verschieden substituiertes Phenyl, wobei die Substituenten aus der Liste W¹ ausgewählt sind.
- Z¹: steht besonders bevorzugt für einfach substituiertes Phenyl, wobei die Substituenten aus der Liste W¹ ausgewählt sind.
- Z¹: steht auch besonders bevorzugt für zweifach, gleich oder verschieden substituiertes Phenyl, wobei die Substituenten aus der Liste W¹ ausgewählt sind.
- Z¹: steht auch besonders bevorzugt für dreifach, gleich oder verschieden substituiertes Phenyl, wobei die Substituenten aus der Liste W¹ ausgewählt sind.
- Z¹: steht ganz besonders bevorzugt für einfach in 4-Position substituiertes Phenyl, wobei die Substituenten aus der Liste W¹ ausgewählt sind.
- Z¹: steht ganz besonders bevorzugt für zweifach, gleich oder verschieden in 3,4-Position substituiertes Phenyl, wobei die Substituenten aus der Liste W¹ ausgewählt sind.
- Z¹: steht ganz besonders bevorzugt für zweifach, gleich oder verschieden in 2,4-Position substituiertes Phenyl, wobei die Substituenten aus der Liste W¹ ausgewählt sind.
- Z¹: steht ganz besonders bevorzugt für zweifach, gleich oder verschieden in 3,5-Position substituiertes Phenyl, wobei die Substituenten aus der Liste W¹ ausgewählt sind.
- Z¹: steht ganz besonders bevorzugt für dreifach, gleich oder verschieden in 2,4,6-Position substituiertes Phenyl, wobei die Substituenten aus der Liste W¹ ausgewählt sind.
- W¹: steht für Halogen, Cyano, Nitro, Amino, Hydroxy, Formyl, Carboxyl, Carbamoyl, Thiocarbamoyl;
jeweils geradkettiges oder verzweigtes Alkyl, Hydroxylalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen; jeweils geradkettiges oder verzweigtes Alkylamino, Dialkylamino, Alkylcarbonyl, Alkylcarbonyloxy, Alkoxycarbonyl, Alkylaminocarbonyl, Dialkylaminocarbonyl, Arylalkylamino-carbonyl, Dialkylaminocarbonyloxy mit 1 bis 6 Kohlenstoffatomen in den jeweiligen Kohlenwasserstoffketten, Alkenylcarbonyl oder Alkinylcarbonyl, mit 2 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung -C(Q¹)=N-Q², worin
Q¹ für Wasserstoff, Hydroxy, Alkyl mit 1 bis 4 Kohlenstoffatomen, Halogenalkyl mit 1 bis 4 Kohlenstoffatomen und 1 bis 9 gleichen oder verschiedenen Halogenatomen oder Cycloalkyl mit 1 bis 6 Kohlenstoffatomen steht und
Q² für Hydroxy, Amino, Methylamino, Phenyl, Benzyl oder für jeweils gegebenenfalls durch Halogen, Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht, sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen.
- W¹: steht bevorzugt für Fluor, Chlor, Brom, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, jeweils zweifach verknüpftes Difluormethylendioxy oder Tetrafluorethylendioxy,
oder eine Gruppierung-C(Q¹)=N-Q², worin
Q¹ für Wasserstoff, Methyl, Ethyl, Trifluormethyl oder Cyclopropyl steht und
Q² für Hydroxy, Methoxy, Ethoxy, Propoxy oder Isopropoxy steht.
- Z und R⁴: stehen auch bevorzugt gemeinsam mit den Kohlenstoffatomen, an die sie gebunden sind, für einen gegebenenfalls einfach bis vierfach, gleich oder verschieden substituierten 5- oder 6-gliedrigen carbocyclischen oder heterocyclischen Ring.
- Z und R⁴: stehen auch besonders bevorzugt gemeinsam mit den Kohlenstoffatomen, an die sie gebunden sind, für einen gegebenenfalls einfach, zweifach oder dreifach durch Methyl substituierten 5- oder 6-gliedrigen carbocyclischen Ring.
- Z und R⁴: stehen auch ganz besonders bevorzugt gemeinsam mit den Kohlenstoffatomen, an die sie gebunden sind, für *-CH(CH₃)-CH₂-C(CH₃)₂-, -(CH₂)₃-, -CH(CH₃)-CH₂-CH(CH₃)-, wobei die mit * markierte Bindung an Z gebunden ist.

Bevorzugt sind außerdem Verbindungen der Formel (Ic) 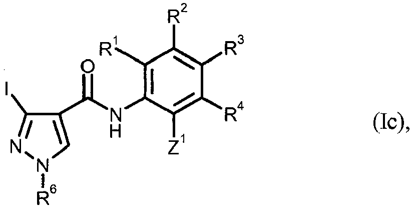 in welcher
R¹, R², R³, R⁴, R⁶ und Z¹ die oben angegebenen Bedeutungen haben.
Besonders bevorzugt sind Verbindungen der Formel (Ic), in welcher R⁶ für Methyl steht.

Bevorzugt sind außerdem Verbindungen der Formel (Ib) 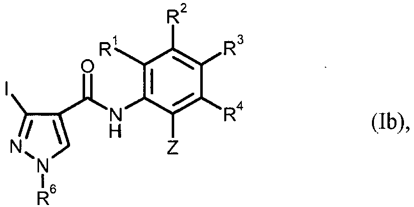 in welcher
R¹, R², R³, R⁴, R⁶ und Z die oben angegebenen Bedeutungen haben.
Besonders bevorzugt sind Verbindungen der Formel (Ib), in welcher R⁶ für Methyl steht.

Bevorzugt sind außerdem Verbindungen der Formel (If) 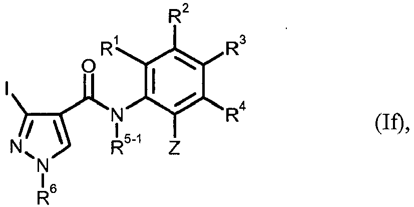 in welcher
- R¹, R², R³, R⁴, R⁵⁻¹, R⁶ und Z: die oben angegebenen Bedeutungen haben.
- R⁵⁻¹: steht bevorzugt für C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₆-Halogencycloalkyl mit jeweils 1 bis 9 Fluor-, Chlor- und/oder Bromatomen, Formyl-C₁-C₃-alkyle, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)-carbonyl-C₁-C₃-halogenalkyl mit jeweils 1 bis 13 Fluor-, Chlor- und/oder Bromatomen; -COR⁷, -CONR⁸R⁹ oder -CH₂NR¹⁰R¹¹.
- R⁵⁻¹: steht besonders bevorzugt für Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec-oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl, -CH₂-CHO, -CH₂CH₂-CHO, -CH₂-CO-CH₃, -CH₂-CO-CH₂CH₃, -CH₂-CO-CH(CH₃)₂, -CH₂CH₂-CO-CH₃, -CH₂CH₂-CO-CH₂CH₃, -CH₂CH₂-CO-CH(CH₃)₂, -CH₂-C(O)OCH₃, -CH₂-C(O)OCH₂CH₃, -CH₂-C(O)OCH(CH₃)₂, -CH₂CH₂-C(O)OCH₃, -CH₂CH₂-C(O)OCH₂CH₃, -CH₂CH₂-C(O)OCH(CH₃)₂, -CH₂-CO-CF₃, -CH₂-CO-CCl₃, -CH₂-CO-CH₂CF₃, -CH₂-CO-CH₂CCl₃, -CH₂CH₂-CO-CH₂CF₃, -CH₂CH₂-CO-CH₂CCl₃, -CH₂-C(O)OCH₂CF₃, -CH₂-C(O)OCF₂CF₃, -CH₂-C(O)OCH₂CCl₃, -CH₂-C(O)OCCl₂CCl₃, -CH₂CH₂-C(O)OCH₂CF₃, -CH₂CH₂-C(O)OCF₂CF₃, -CH₂CH₂-C(O)OCH₂CCl₃, -CH₂CH₂-C(O)O-CCl₂CCl₃; -COR⁷, -CONR⁸R⁹ oder -CH₂NR¹⁰R¹¹.
- R⁵⁻¹: steht ganz besonders bevorzugt für Methyl, Methoxymethyl, -CH₂-CHO, -CH₂CH₂-CHO, -CH₂-CO-CH₃, -CH₂-CO-CH₂CH₃, -CH₂-CO-CH(CH₃)₂ oder -COR⁷.
Besonders bevorzugt sind Verbindungen der Formel (If), in welcher R⁶ für Methyl steht.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Die Definition C₁-C₂₀-Alkyl umfasst den größten hierin definierten Bereich für einen Alkylrest. Im Einzelnen umfasst diese Definition die Bedeutungen Methyl, Ethyl, n-, iso-Propyl, n-, iso-, sec-, tert-Butyl, sowie jeweils alle isomeren Pentyle, Hexyle, Heptyle, Octyle, Nonyle, Decyle, Undecyle, Dodecyle, Tridecyle, Tetradecyle, Pentadecyle, Hexadecyle, Heptadecyle, Octadecyle, Nonadecyle und Eicosyle. Hierunter bevorzugt sind die Bedeutungen Methyl, Ethyl, n-, iso-Propyl, n-, iso-, sec-, tert-Butyl, n-Pentyl, 1-Methylbutyl, 2-Methylbutyl, 3-Methylbutyl, 1,2-Dimethylpropyl, 2,2-Dimethylpropyl, n-Hexyl, 1-Methylpentyl, 4-Methylpentyl, 1,3-Dimethylbutyl, 3,3-Dimethylbutyl, 1,2,2-Trimethylpropyl, n-Heptyl, 1-Methylhexyl, 5-Methylhexyl, 1,4-Dimethylpentyl, 4,4-Dimethylpentyl, 1,3,3-Trimethylbutyl, 1,2,3-Trimethylbutyl.

Die Definition C₂-C₂₀-Alkenyl umfasst den größten hierin definierten Bereich für einen Alkenylrest. Im Einzelnen umfasst diese Definition die Bedeutungen Ethenyl, 1-Propenyl, 2-Propenyl, isoPropenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Ethylethenyl, sowie jeweils alle isomeren Pentenyle, Hexenyle, Heptenyle, Octenyle, Nonenyle, Decenyle, Undecenyle, Dodecenyle, Tridecenyle, Tetradecenyle, Pentadecenyle, Hexadecenyle, Heptadecenyle, Octadecenyle, Nonadecenyle und Eicosenyle. Hierunter bevorzugt sind die Bedeutungen Ethenyl, 1-Propenyl, 2-Propenyl, 1-Butenyl, 2-Butenyl, 3-Butenyl, 1-Methyl-1-propenyl, 2-Methyl-1-propenyl, 1-Pentenyl, 4-Pentenyl, 1-Methyl-1-butenyl, 1,2-Dimethyl-1-propenyl, 1-Hexenyl, 5-Hexenyl, 1-Methyl-1-pentenyl, 1,3-Dimethyl-1-butenyl, 1-Methyl-1-hexenyl, 1,3,3-Trimethyl-1-butenyl.

Die Definition C₂-C₂₀-Alkinyl umfasst den größten hierin definierten Bereich für einen Alkinylrest. Im Einzelnen umfasst diese Definition die Bedeutungen Ethinyl, 1-Propinyl, 2-Propinyl, 1-Butinyl, 2-Butinyl, 3-Butinyl, 1-Methyl-2-propinyl, sowie jeweils alle isomeren Pentinyle, Hexinyle, Heptinyle, Octinyle, Noninyle, Decinyle, Undecinyle, Dodecinyle, Tridecinyle, Tetradecinyle, Pentadecinyle, Hexadecinyle, Heptadecinyle, Octadecinyle, Nonadecinyle und Eicosinyle. Hierunter bevorzugt sind die Bedeutungen Ethinyl, 1-Propinyl, 1-Butinyl, 3-Butinyl, 1-Methyl-2-propinyl, 1-Pentinyl, 4-Pentinyl, 1-Hexinyl, 5-Hexinyl, 3,3-Dimethyl-1-butinyl, 4,4-Dimethyl-1-pentinyl, 4,4-Dimethyl-2-pentinyl, 1,4-Dimethyl-2-pentinyl.

Gegebenenfalls substituierte Reste können einfach oder mehrfach substituiert sein, wobei bei Mehrfachsubstitutionen die Substituenten gleich oder verschieden sein können.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können zwischen den jeweiligen Bereichen und Vorzugsbereichen beliebig kombiniert werden. Sie gelten für die Endprodukte sowie für die Vor- und Zwischenprodukte entsprechend.

### Erläuterungen der Verfahren und Zwischenprodukte:

### Verfahren (a)

Verwendet man 3-Iod-1-methyl-1*H*-pyrazol-4-carbonsäure und 3',4'-Dichlor-5-fluor-1,1'-biphenyl-2-amin als Ausgangsstoffe, so kann der Verlauf des erfindungsgemäßen Verfahrens (a) durch das folgende Formelschema veranschaulicht werden.

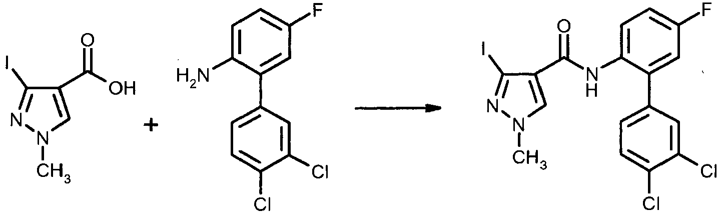

Die zur Durchführung des erfindungsgemäßen Verfahrens (a) als Ausgangsstoffe benötigten Iodpyrazolylcarbonsäurederivate sind durch die Formel (II) allgemein definiert. In dieser Formel (II) steht X¹ bevorzugt für Chlor oder Hydroxy.

Die Iodpyrazolylcarbonsäurederivate der Formel (II) sind teilweise bekannt. Sie werden erhalten, indem man
j) 3-Amino-pyrazol-4-carbonsäureester der Formel (XIV) 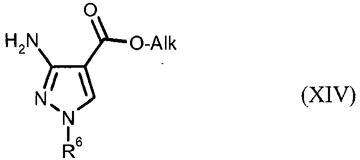 in welcher
   - Alk: für C₁-C₄-Alkyl steht und
   - R⁶: die oben angegebenen Bedeutungen hat,
   in einem ersten Schritt mit einem Iodierungsmittel (z.B. Methyleniodid) in Gegenwart von Isoamylnitrit umsetzt
   und die so erhaltenen 3-Iod-pyrazol-4-carbonsäureester der Formel (XV) 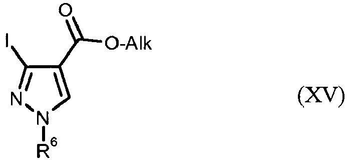 in welcher Alk und R⁶ die oben angegebenen Bedeutungen haben,
   in einem zweiten Schritt mit einer Base (z.B. NaOH oder KOH) in Gegenwart eines Verdünnungsmittels (z.B. Ethanol) zur Säure verseift [Verbindungen der Formel (II), in denen X¹ für Hydroxy steht]
   und diese Säure in einem dritten Schritt gegebenenfalls mit einem Chlorierungsmittel (z.B. Thionylchlorid/Oxalylchlorid) in Gegenwart eines Verdünnungsmittels (z.B. Toluol oder Methylenchlorid) zum entsprechenden Säurechlorid umsetzt [Verbindungen der Formel (II), in denen X¹ für Chlor steht].

In den Verbindungen der Formel (XIV) und (XV) hat R⁶ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Verbindungen der Formeln (XIV) und (XV) sind teilweise bekannt (vgl. WO 93/11117, JP 2002-128763). 3-Amino-pyrazol-4-carbonsäureester der Formel (XIV) werden außerdem erhalten, indem man
k) Benzylidenhydrazin-Derivate der Formel (XVI) 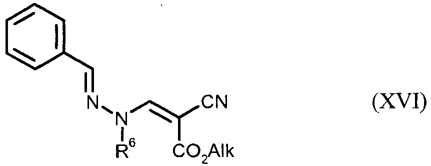 in welcher R⁶ und Alk die oben angegebenen Bedeutungen haben,
   in Gegenwart einer Säure (z.B. HCl) und in Gegenwart eines Verdünnungsmittels (z.B. Ethanol) cyclisiert.

In den Verbindungen der Formel (XVI) hat R⁶ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Verbindungen der Formel (XVI) sind neu. Sie werden erhalten, indem man
l) Benzylidenhydrazine der Formel (XVII) 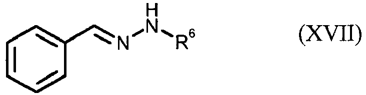 in welcher R⁶ die oben angegebenen Bedeutungen hat,
   mit Cyanessigsäureestern der Formel (XVIII) 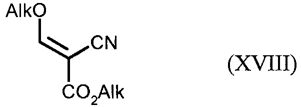 in welcher Alk die oben angegebenen Bedeutungen hat,
   in Gegenwart eines Verdünnungsmittels (z.B. Toluol) umsetzt (vgl. J. Org. Chem. 1983, 48, 4116-4119).

Die Benzylidenhydrazine der Formel (XVII) und die Cyanessigsäureester der Formel (XVIII) sind bekannt und/oder können nach bekannten Methoden hergestellt werden.

Die zur Durchführung des erfindungsgemäßen Verfahrens (a) weiterhin als Ausgangsstoffe benötigten Anilin-Derivate sind durch die Formel (III) allgemein definiert. In dieser Formel (III) haben R¹, R², R³, R⁴, R⁵ und Z bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Ausgangsstoffe der Formel (III) sind größtenteils bekannt und/oder können nach bekannten Verfahren hergestellt werden (vgl. z.B. Bull. Korean Chem. Soc. 2000, 21, 165-166; Chem. Pharm. Bull. 1992, 40, 240-244; Heterocycles 1989, 29, 1013-1016; J. Med. Chem. 1996, 39, 892-903; Synthesis 1995, 713-16; Synth. Commun. 1994, 24, 267-272; Synthesis 1994, 142-144; DE-A 27 27 416; DE-A 102 190 35; JP-A 9-132567; EP-A 0 824 099; WO 93/11117; EP-A 0 545 099; EP-A 0 589 301; EP-A 0 589 313 und WO 02/38542).

Es ist auch möglich zunächst Anilin-Derivate der Formel (III), in welcher R⁵ für Wasserstoff steht, herzustellen und die so erhaltenen Verbindungen anschließend nach üblichen Methoden zu derivatisieren (z.B. analog zum erfindungsgemäßen Verfahren (i)).

### Verfahren (b)

Verwendet man *N*-(2-Bromphenyl)-3-iod-1-methyl-1*H*-pyrazol-4-carboxamid und 4-Chlor-3-fluorphenylboronsäure als Ausgangsstoffe sowie einen Katalysator, so kann der Verlauf des erfindungsgemäßen Verfahrens (b) durch das folgende Formelschema veranschaulicht werden.

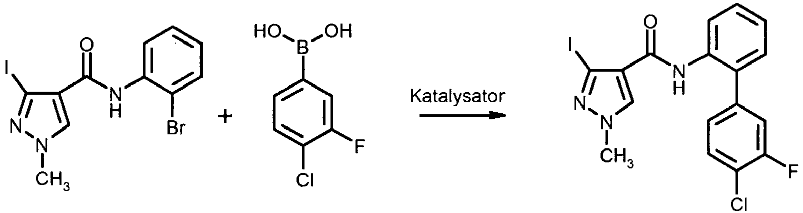

Die zur Durchführung des erfindungsgemäßen Verfahrens (b) als Ausgangsstoffe benötigten Halogen-iodpyrazolylcarboxanilide sind durch die Formel (IV) allgemein definiert. In dieser Formel (IV) haben R¹, R², R³, R⁴, R⁵ und R⁶ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden. X² steht für Brom oder Iod.

Die Halogen-iodpyrazolylcarboxanilide der Formel (IV) sind noch nicht bekannt. Sie sind als neue chemische Verbindungen ebenfalls Gegenstand der vorliegenden Anmeldung. Sie werden erhalten, indem man
m) Iodpyrazolylcarbonsäurederivate der Formel (II) 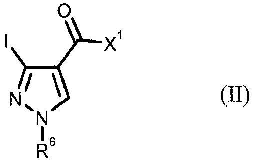 in welcher
   - X¹: für Chlor oder Hydroxy steht,
   - R⁶: die oben angegebenen Bedeutungen hat,
   mit Halogenanilinen der Formel (XIX), 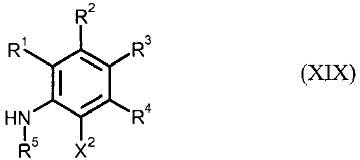 in welcher R¹, R², R³, R⁴, R⁵ und X² die oben angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

### Verfahren (m)

Verwendet man 3-Iod-1-methyl-1H-pyrazol-4-carbonsäurechlorid und 2-Bromanilin als Ausgangsstoffe, so kann der Verlauf des erfindungsgemäßen Verfahrens (m) durch das folgende Formelschema veranschaulicht werden.

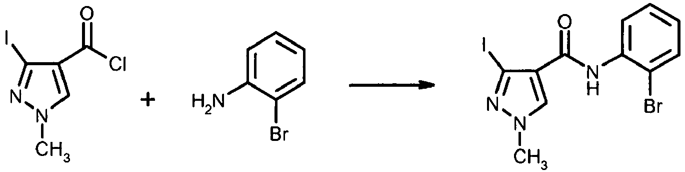

Die zur Durchführung des erfindungsgemäßen Verfahrens (m) als Ausgangsstoffe benötigten Iodpyrazolylcarbonsäurederivate der Formel (II) sind bereits weiter oben im Zusammenhang mit dem erfindungsgemäßen Verfahren (a) beschrieben worden.

Die zur Durchführung des erfindungsgemäßen Verfahrens (m) weiterhin als Ausgangsstoffe benötigten Halogenaniline sind durch die Formel (XIX) allgemein definiert. In dieser Formel (XIX) haben R¹, R², R³, R⁴, R⁵ und X² bevorzugt, besonders bevorzugt bzw. ganz besonders bevorzugt diejenigen Bedeutungen, die bereits im Zusammenhang mit der Beschreibung der erfindungsgemäßen Verbindungen der Formel (I), bzw. die Vorprodukte der Formel (III) als bevorzugt, besonders bevorzugt bzw. ganz besonders bevorzugt für diese Reste angegeben wurden.

Die Halogenaniline der Formel (XIX) sind bekannte Synthesechemikalien oder können nach bekannten Verfahren erhalten werden. Im Fall, dass R⁵ nicht für Wasserstoff steht, kann der Rest R⁵ auf der Stufe der Verbindungen der Formel (XIX) durch übliche Derivatisierungsmethoden eingeführt werden. Es ist auch möglich, zunächst Verbindungen der Formel (IV), in denen R⁵ für Wasserstoff steht, herzustellen und die erhaltenen Produkte anschließend durch übliche Methoden zu derivatisieren (vgl. das erfindungsgemäße Verfahren (i)).

Die zur Durchführung des erfindungsgemäßen Verfahrens (b) weiterhin als Ausgangsstoffe benötigten Boronsäure-Derivate sind durch die Formel (V) allgemein definiert. In dieser Formel (V) hat Z¹ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für Z¹ angegeben wurden. A¹ und A² stehen jeweils für Wasserstoff oder zusammen für Tetramethylethylen.

Die Boronsäure-Derivate der Formel (V) sind bekannt und/oder lassen sich nach bekannten Verfahren herstellen (vgl. z.B. WO 01/90084 und US 5,633,218).

### Verfahren (c)

Verwendet man 2-{[(3-Iod-1-methyl-1*H*-pyrazol-4-yl)carbonyl]amino}phenylboronsäure und 1-Brom-4-chlor-3-fluorbenzol als Ausgangsstoffe sowie einen Katalysator, so kann der Verlauf des erfindungsgemäßen Verfahrens (c) durch das folgende Formelschema veranschaulicht werden.

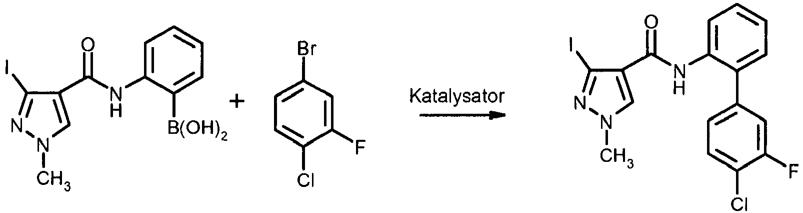

Die zur Durchführung des erfindungsgemäßen Verfahrens (c) als Ausgangsstoffe benötigten Iodpyrazolylcarboxamid-Boronsäure-Derivate sind durch die Formel (VI) allgemein definiert. In dieser Formel (VI) haben R¹, R², R³, R⁴, R⁵ und R⁶ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden. A³ und A⁴ stehen jeweils für Wasserstoff oder zusammen für Tetramethylethylen.

Die Iodpyrazolylcarboxamid-Boronsäure-Derivate der Formel (VI) sind noch nicht bekannt. Sie sind neue chemische Verbindungen und ebenfalls Gegenstand der vorliegenden Anmeldung. Sie werden erhalten, indem man
n) Iodpyrazolylcarbonsäurederivate der Formel (II) 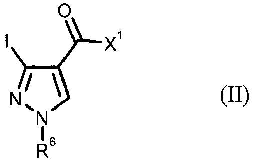 in welcher
   - X¹: für Chlor oder Hydroxy steht,
   - R⁶: die oben angegebenen Bedeutungen hat,
   mit Anilinboronsäurederivaten der Formel (XX) 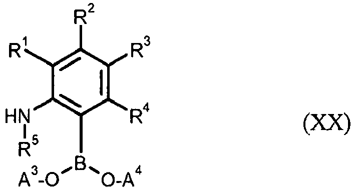 in welcher
   R¹, R², R³, R⁴, R⁵, A³ und A⁴ die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

### Verfahren (n)

Verwendet man 3-Iod-1-methyl-1*H*-pyrazol-4-carbonsäurechlorid und 2-Aminophenylboronsäure als Ausgangsstoffe, so kann der Verlauf des erfindungsgemäßen Verfahrens (n) durch das folgende Formelschema veranschaulicht werden.

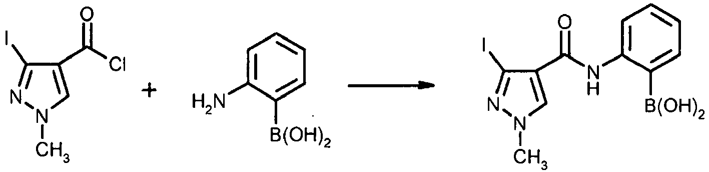

Die zur Durchführung des erfindungsgemäßen Verfahrens (n) als Ausgangsstoffe benötigten Iodpyrazolylcarbonsäurederivate der Formel (II) sind bereits weiter oben im Zusammenhang mit dem erfindungsgemäßen Verfahren (a) beschrieben worden.

Die weiterhin zur Durchführung des erfindungsgemäßen Verfahrens (n) als Ausgangsstoffe benötigten Anilinboronsäurederivate sind durch die Formel (XX) allgemein definiert. In dieser Formel (XX) haben R¹, R², R³, R⁴, R⁵ und R⁶ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden. A³ und A⁴ stehen jeweils für Wasserstoff oder zusammen für Tetramethylethylen.

Die Anilinboronsäurederivate der Formel (XX) sind bekannte Synthesechemikalien oder können nach bekannten Verfahren erhalten werden. Im Fall, dass R⁵ nicht für Wasserstoff steht, kann der Rest R⁵ auf der Stufe der Verbindungen der Formel (XX) durch übliche Derivatisierungsmethoden eingeführt werden. Es ist auch möglich, zunächst Verbindungen der Formel (VI), in denen R⁵ für Wasserstoff steht, herzustellen und die erhaltenen Produkte anschließend durch übliche Methoden zu derivatisieren (vgl. das erfindungsgemäße Verfahren (i)).

Die zur Durchführung des erfindungsgemäßen Verfahrens (c) weiterhin als Ausgangsstoffe benötigten Phenyl-Derivate sind durch die Formel (VII) allgemein definiert. In dieser Formel (VII) hat Z¹ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für Z¹ angegeben wurden. X³ steht für Chlor, Brom, Iod oder Trifluormethylsulfonat.

Die Phenyl-Derivate der Formel (VII) sind bekannte Synthesechemikalien.

### Verfahren (d)

Verwendet man *N*-(2-Bromphenyl)-3-iod-1-methyl-1*H*-pyrazol-4-carboxamid und 1-Brom-4-chlor-3-fluorbenzol als Ausgangsstoffe sowie einen Katalysator und 4,4,4',4',5,5,5',5'-Octamethyl-2,2'-bis-1,3,2-dioxaborolan, so kann der Verlauf des erfindungsgemäßen Verfahrens (d) durch das folgende Formelschema veranschaulicht werden.

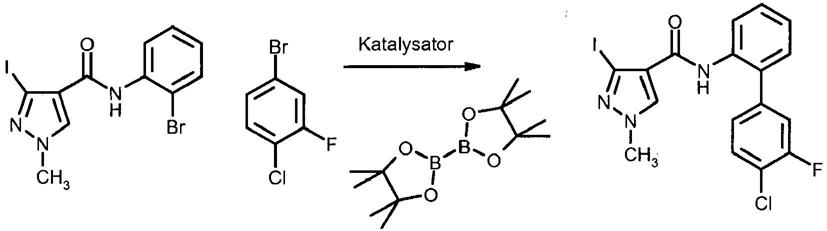

Die zur Durchführung des erfindungsgemäßen Verfahrens (d) als Ausgangsstoffe benötigten Halogen-iodpyrazolylcarboxanilide der Formel (IV), sowie die Phenyl-Derivate der Formel (VII) sind bereits weiter oben im Zusammenhang mit den erfindungsgemäßen Verfahren (b) und (c) beschrieben worden.

Das weiterhin zur Durchführung des erfindungsgemäßen Verfahrens (d) benötigte 4,4,4',4',5,5,5',5'- Octamethyl-2,2'-bis-1,3,2-dioxaborolan ist eine handelsübliche Synthesechemikalie.

### Verfahren (e)

Hydriert man beispielsweise *N*-{2-[1,3-Dimethyl-1-butenyl]phenyl}-3-iod-1-methyl-1*H*-pyrazol-4-carboxamid, so kann der Verlauf des erfindungsgemäßen Verfahrens (e) durch das folgende Formelschema veranschaulicht werden.

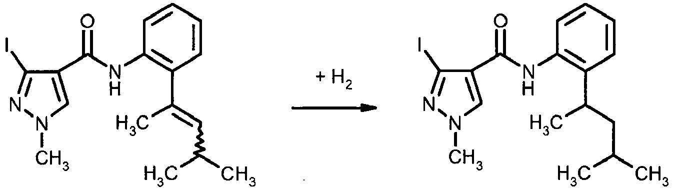

Die zur Durchführung des erfindungsgemäßen Verfahrens (e) als Ausgangsstoffe benötigten Iodpyrazolylcarboxanilide sind durch die Formel (Ia) allgemein definiert. In dieser Formel (Ia) haben R¹, R², R³, R⁴, R⁵ und R⁶ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Verbindungen der Formel (Ia) sind erfindungsgemäße Verbindungen und können nach den Verfahren (a), (f), (g) oder (h) hergestellt werden.

### Verfahren (f)

Dehydratisiert man beispielsweise *N*-[2-(1-Hydroxy-1,3-dimethylbutyl)phenyl]-3-iod-1-methyl-1*H-*pyrazol-4-carboxamid, so kann der Verlauf des erfindungsgemäßen Verfahrens (f) durch das folgende Formelschema veranschaulicht werden.

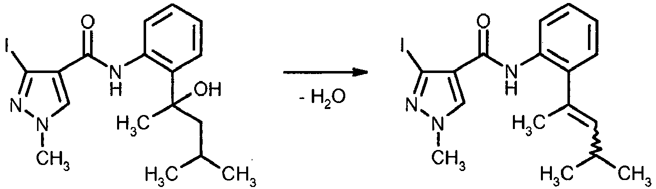

Die zur Durchführung des erfindungsgemäßen Verfahrens (f) als Ausgangsstoffe benötigten Hydroxyalkyl-iodpyrazolylcarboxanilide sind durch die Formel (VIII) allgemein definiert. In dieser Formel (VIII) haben R¹, R², R³, R⁴, R⁵ und R⁶ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.
- X⁵: steht bevorzugt für gegebenenfalls zusätzlich einfach bis vierfach, gleich oder verschieden durch Chlor, Fluor, Brom und/oder C₃-C₆-Cycloalkyl substituiertes C₂-C₁₂- Hydroxyalkyl, wobei der Cycloalkylteil seinerseits gegebenenfalls durch Halogen und/oder C₁-C₄-Alkyl substituiert sein kann.
- X⁵: steht besonders bevorzugt für gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Hydroxyethyl, Hydroxypropyl, Hydroxybutyl, Hydroxypentyl, Hydroxyhexyl, Hydroxyheptyl, Hydroxyoctyl, Hydroxynonyl oder Hydroxydecyl.

Die Verbindungen der Formel (VIII) sind noch nicht bekannt und als neue Verbindungen ebenfalls Gegenstand der vorliegenden Anmeldung.
Es wurde auch gefunden, dass die Hydroxyalkyl-iodpyrazolylcarboxanilide der Formel (VIII) sehr gute mikrobizide Eigenschaften besitzen und zur Bekämpfung unerwünschter Mikroorganismen sowohl im Pflanzenschutz als auch im Materialschutz eingesetzt werden können.

Die Hydroxyalkyl-iodpyrazolylcarboxanilide der Formel (VIII) werden erhalten, indem man
o) Iodpyrazolylcarbonsäurederivate der Formel (II) 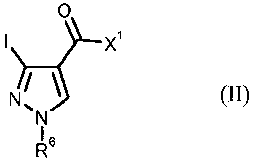 in welcher
   - X¹: für Chlor oder Hydroxy steht,
   - R⁶: die oben angegebenen Bedeutungen hat,
   mit Hydroxyalkylanilinderivaten der Formel (XXI) 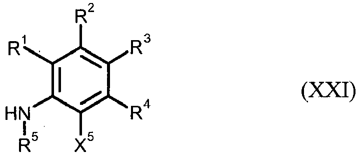 in welcher
   R¹, R², R³, R⁴, R⁵ und X⁵ die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

### Verfahren (o)

Verwendet man beispielsweise 3-Iod-1-methyl-1*H*-pyrazol-4-carbonsäurechlorid carbonsäurechlorid und 2-(2-Aminophenyl)-2-pentanol als Ausgangsstoffe, so kann der Verlauf des erfindungsgemäßen Verfahrens (o) durch das folgende Formelschema veranschaulicht werden:

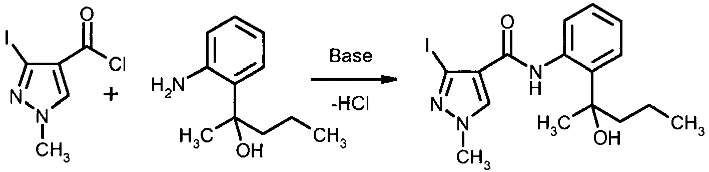

Die zur Durchführung des erfindungsgemäßen Verfahrens (o) als Ausgangsstoffe benötigten Iodpyrazolylcarbonsäurederivate der Formel (II) sind bereits weiter oben im Zusammenhang mit dem erfindungsgemäßen Verfahren (a) beschrieben worden.

Die zur Durchführung des erfindungsgemäßen Verfahrens (o) als Ausgangsstoffe weiterhin benötigten Hydroxyalkylanilinderivate sind durch die Formel (XXI) allgemein definiert. In dieser Formel (XXI) haben R¹, R², R³, R⁴, R⁵ und X⁵ bevorzugt, besonders bevorzugt bzw. ganz besonders bevorzugt diejenigen Bedeutungen, die bereits im Zusammenhang mit der Beschreibung der erfindungsgemäßen Verbindungen der Formeln (I) bzw. (VIII) als bevorzugt, besonders bevorzugt bzw. ganz besonders bevorzugt für diese Reste angegeben wurden.

Die Hydroxyalkylanilinderivate der Formel (XXI) sind bekannt und/oder können nach bekannten Methoden erhalten werden (vgl. z.B. US 3,917,592 oder EP-A 0 824 099). Im Fall, dass R⁵ nicht für Wasserstoff steht, kann der Rest R⁵ auf der Stufe der Verbindungen der Formel (XXI) durch übliche Derivatisierungsmethoden eingeführt werden. Es ist auch möglich, zunächst Verbindungen der Formel (VIII), in denen R⁵ für Wasserstoff steht, herzustellen und die erhaltenen Produkte anschlieβend durch übliche Methoden zu derivatisieren (vgl. das erfindungsgemäße Verfahren (i)).

### Verfahren (g)

Verwendet man beispielsweise *N*-(2-Bromphenyl)-3-iod-1-methyl-1*H*-pyrazol-4-carboxamid und 1-Hexin als Ausgangsstoffe sowie einen Katalysator, so kann der Verlauf des erfindungsgemäßen Verfahrens (g) durch das folgende Formelschema veranschaulicht werden.

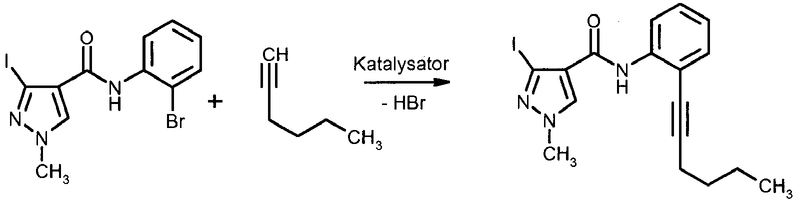

Die zur Durchführung des erfindungsgemäßen Verfahrens (g) als Ausgangsstoffe benötigten Halogen-iodpyrazolylcarboxanilide der Formel (IV) sind bereits weiter oben im Zusammenhang mit dem erfindungsgemäßen Verfahrens (c) beschrieben worden.

Die zur Durchführung des erfindungsgemäßen Verfahrens (g) weiterhin als Ausgangsstoffe benötigten Alkine sind durch die Formel (IX) allgemein definiert.
- A⁵: steht bevorzugt für gegebenenfalls einfach bis vierfach, gleich oder verschieden durch Fluor, Chlor, Brom und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- A⁵: steht besonders bevorzugt für gegebenenfalls einfach bis vierfach, gleich oder verschieden durch Fluor, Chlor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.

Die Alkine der Formel (IX) sind bekannte Synthesechemikalien.

Die zur Durchführung des erfindungsgemäßen Verfahrens (g) weiterhin alternativ als Ausgangsstoffe benötigten Alkene sind durch die Formel (X) allgemein definiert.
- A⁶, A⁷ und A⁸: stehen unabhängig voneinander bevorzugt jeweils für Wasserstoff oder gegebenenfalls einfach bis vierfach, gleich oder verschieden durch Fluor, Chlor, Brom und/oder C₃-C₆-Cycloalkyl substituiertes Alkyl, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils die Zahl 12 nicht übersteigt.
- A⁶, A⁷ und A⁸: stehen unabhängig voneinander besonders bevorzugt jeweils für Wasserstoff oder gegebenenfalls einfach bis vierfach, gleich oder verschieden durch Fluor, Cyclopropyl, Difluorcyclopropyl, Cyclobutyl, Cyclopentyl und/ oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl, wobei die Gesamtzahl der Kohlenstoffatome des offenkettigen Molekülteils die Zahl 12 nicht übersteigt.

Die Alkene der Formel (X) sind bekannte Synthesechemikalien.

### Verfahren (h)

Verwendet man N-(2-Acetylphenyl)-3-iod-1-methyl-1H-pyrazol-4-carboxamid und Butyl(triphenyl)-phosphonium-iodid als Ausgangsstoffe, so kann der Verlauf des erfindungsgemäßen Verfahrens (h) durch das folgende Formelschema veranschaulicht werden:

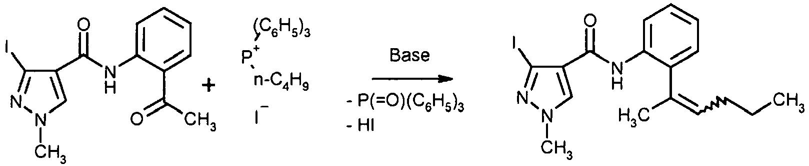

Die zur Durchführung des erfindungsgemäßen Verfahrens (h) als Ausgangsstoffe benötigten Ketone sind durch die Formel (XI) allgemein definiert. In dieser Formel haben R¹, R², R³, R⁴, R⁵ und R⁶ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.
- A⁹: steht bevorzugt für gegebenenfalls einfach bis vierfach, gleich oder verschieden durch Fluor, Chlor, Brom und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- A⁹: steht besonders bevorzugt für gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.

Die Ketone der Formel (XI) sind noch nicht bekannt. Sie sind als neue chemische Verbindungen ebenfalls Gegenstand der vorliegenden Anmeldung. Sie werden erhalten, indem man
p) Iodpyrazolylcarbonsäurederivate der Formel (II) 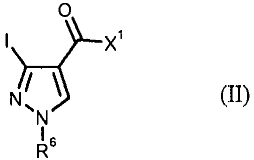 in welcher
   - X¹: für Chlor oder Hydroxy steht,
   - R⁶: die oben angegebenen Bedeutungen hat,
   mit Ketoanilinen der Formel (XXII) 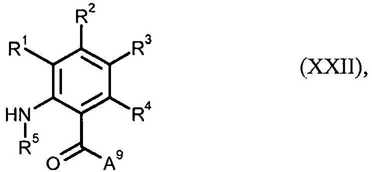 in welcher R¹, R², R³, R⁴, R⁵ und A⁹ die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

### Verfahren (p)

Verwendet man 3- Iod-1-methyl-1H-pyrazol-4-carbonsäurechlorid und 1-(2-Aminophenyl)ethanon als Ausgangsstoffe, so kann der Verlauf des erfindungsgemäßen Verfahrens (p) durch das folgende Formelschema veranschaulicht werden:

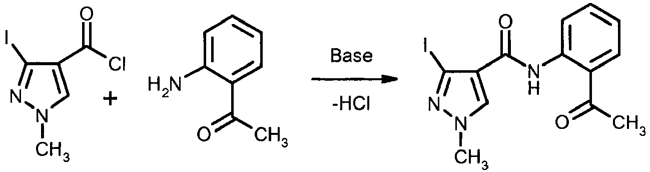

Die zur Durchführung des erfindungsgemäßen Verfahrens (p) als Ausgangsstoffe benötigten Iodpyrazolylcarbonsäurederivate der Formel (II) sind bereits weiter oben im Zusammenhang mit dem erfindungsgemäßen Verfahrens (a) beschrieben worden.

Die zur Durchführung des erfindungsgemäßen Verfahrens (p) weiterhin als Ausgangsstoffe benötigten Ketoaniline sind durch die Formel (XXII) allgemein definiert. In dieser Formel (XXII) haben R¹, R², R³, R⁴, R⁵ und A⁹ bevorzugt, besonders bevorzugt bzw. ganz besonders bevorzugt diejenigen Bedeutungen, die bereits im Zusammenhang mit der Beschreibung der erfindungsgemäßen Verbindingen der Formeln (I) bzw. (XI) als bevorzugt, besonders bevorzugt bzw. ganz besonders bevorzugt für diese Reste angegeben wurden.

Die Ketoaniline der Formel (XXII) sind bekannt (vgl. J. Am. Chem. Soc. 1978, 100, 4842-4857 oder US 4,032,573) und/oder können nach bekannten Methoden erhalten werden. Im Fall, dass R⁵ nicht für Wasserstoff steht, kann der Rest R⁵ auf der Stufe der Verbindungen der Formel (XXII) durch übliche Derivatisierungsmethoden eingeführt werden. Es ist auch möglich, zunächst Verbindungen der Formel (VIII), in denen R⁵ für Wasserstoff steht, herzustellen und die erhaltenen Produkte anschließend durch übliche Methoden zu derivatisieren (vgl. das erfindungsgemäße Verfahren (i)).

Die zur Durchführung des erfindungsgemäßen Verfahrens (h) weiterhin als Ausgangsstoffe benötigten Phosphorverbindungen sind durch die Formel (XII) allgemein definiert.
- A¹⁰: steht bevorzugt für gegebenenfalls einfach bis vierfach, gleich oder verschieden durch Chlor, Fluor, Brom und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- A¹⁰: steht besonders bevorzugt für gegebenenfalls einfach bis vierfach, gleich oder verschieden durch Fluor, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.
- Px: steht bevorzugt für eine Gruppierung -P⁺(C₆H₅)₃Cl⁻, -P⁺(C₆H₅)₃ Br⁻, -P⁺(C₆H₅)₃ I⁻, -P(=O)(OCH₃)₃ oder -P(=O)(OC₂H₅)₃.

Die Phosphorverbindungen der Formel (XII) sind bekannt und/oder können nach bekannten Verfahren hergestellt werden (vgl. Justus Liebigs Ann. Chem. 1953, 580, 44-57 oder Pure Appl. Chem. 1964, 9, 307-335).

### Verfahren (i)

Verwendet man *N*-(4'-Chlor-3'-fluor-1,1'-biphenyl-2-yl)-3-iod-1-methyl-1H-pyrazol-4-carboxamid und Acetylchlorid als Ausgangsstoffe, so kann der Verlauf des erfindungsgemäßen Verfahrens (i) durch das folgende Formelschema veranschaulicht werden:

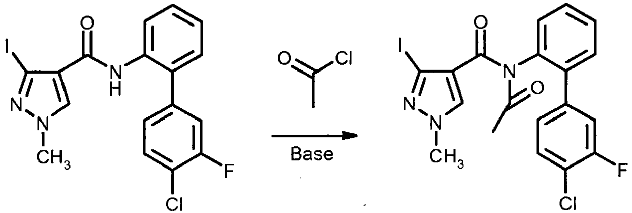

Die zur Durchführung des erfindungsgemäßen Verfahrens (i) als Ausgangsstoffe benötigten Iodpyrazolylcarboxanilide sind durch die Formel (Ib) allgemein definiert. In dieser Formel (Ib) haben R¹, R², R³, R⁴, R⁶ und Z bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Verbindungen der Formel (Ib) sind erfindungsgemäße Verbindungen und können nach den Verfahren (a) bis (h) hergestellt werden.

Die zur Durchführung des erfindungsgemäßen Verfahrens (i) weiterhin als Ausgangsstoffe benötigten Halogenide sind durch die Formel (XIII) allgemein definiert. In dieser Formel (XIII) steht R⁵⁻¹ bevorzugt, besonders bevorzugt bzw. ganz besonders bevorzugt für diejenigen Bedeutungen, die bereits oben in Zusammenhang mit der Beschreibung der Verbindungen der Formel (Ig) als bevorzugt, besonders bevorzugt bzw. ganz besonders bevorzugt für diese Reste angegeben wurden. X⁶ steht für Chlor, Brom oder Iod.

Halogenide der Formel (XIII) sind bekannt.

### Reaktionsbedingungen

Als Verdünnungsmittel zur Durchführung der erfindungsgemäßen Verfahren (a), (m), (n), (o) und (p) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isöbutyllceton oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylfonnanilid, N-Methylpyrrolidon oder Hexamethylphosphorsäuretriamid; deren Gemische mit Wasser oder reines Wasser.

Die erfindungsgemäßen Verfahren (a), (m), (n), (o) und (p) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren (a), (m), (n), (o) und (p) werden gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ), Triphenylphosphin/ Tetrachlorkohlenstoff oder Brom-tripyrrolidinophosphonium-hexafluorophosphat.

Die erfindungsgemäßen Verfahren (a), (m), (n), (o) und (p) werden gegebenenfalls in Gegenwart eines Katalysators durchgeführt. Beispielsweise genannt seien 4-Dimethylaminopyridin, 1-Hydroxybenzotriazol oder Dimethylformamid.

Die Reaktionstemperaturen können bei der Durchführung der erfindungsgemäßen Verfahren (a), (m), (n), (o) und (p) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens (a) zur Herstellung der Verbindungen der Formel (I) setzt man pro Mol des Iodpyrazolylcarbonsäurederivates der Formel (II) im Allgemeinen 0,8 bis 15 Mol, vorzugsweise 0,8 bis 8 Mol an Anilin-Derivat der Formel (III) ein.

Zur Durchführung des erfindungsgemäßen Verfahrens (j) zur Herstellung der Verbindungen der Formel (IV) setzt man pro Mol des Iodpyrazolylcarbonsäurederivates der Formel (II) im Allgemeinen 0,8 bis 15 Mol, vorzugsweise 0,8 bis 8 Mol an Halogenaniline der Formel (XIII) ein.

Zur Durchführung des erfindungsgemäßen Verfahrens (k) zur Herstellung der Verbindungen der Formel (VI) setzt man pro Mol des Iodpyrazolylcarbonsäurederivates der Formel (II) im Allgemeinen 0,8 bis 15 Mol, vorzugsweise 0,8 bis 8 Mol an Anilinboronsäurederivat der Formel (XIV) ein.

Zur Durchführung des erfindungsgemäßen Verfahrens (l) zur Herstellung der Verbindungen der Formel (VIII) setzt man pro Mol des Iodpyrazolylcarbonsäurederivates der Formel (II) im Allgemeinen 0,8 bis 15 Mol, vorzugsweise 0,8 bis 8 Mol an Hydroxyalkylanilinderivat der Formel (XV) ein.

Zur Durchführung des erfindungsgemäßen Verfahrens (m) zur Herstellung der Verbindungen der Formel (IX) setzt man pro Mol des Iodpyrazolylcarbonsäurederivates der Formel (II) im Allgemeinen 0,8 bis 15 Mol, vorzugsweise 0,8 bis 8 Mol an Ketoanilin der Formel (XVI) ein.

Als Verdünnungsmittel zur Durchführung der erfindungsgemäßen Verfahren (b), (c) und (d) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N.N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung der erfindungsgemäßen Verfahren (b), (c) und (d) in einem größeren Bereich variiert werden. Im allgemeinen arbeitet man bei Temperaturen von 0°C bis 180°C, vorzugsweise bei Temperaturen von 20°C bis 150°C.

Die der erfindungsgemäßen Verfahren (b), (c) und (d) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, fluoride, phosphate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumphosphat, Kaliumphosphat, Kaliumfluorid, Cäsiumfluorid, Natriumcarbonat, Kaliumcarbonat, Kaliumhydrogencarbonat, Natriumhydrogencarbonat oder Cäsiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die der erfindungsgemäßen Verfahren (b), (c) und (d) werden in Gegenwart eines Katalysators, wie beispielsweise eines Palladiumsalzes oder -komplexes, durchgeführt. Hierzu kommen vorzugsweise Palladiumchlorid, Palladiumacetat, Tetrakis-(triphenylphosphin)-Palladium, Bis-(triphenylphosphin)-Palladiumdichlorid oder (1,1'-Bis(diphenylphosphino)ferrocenpalladium(II)chlorid) infrage.

Es kann auch ein Palladiumkomplex in der Reaktionsmischung erzeugt werden, wenn man ein Palladiumsalz und ein Komplexligand, wie z.B. Triethylphosphan, Tri-tert-butylphosphan, Tricyclohexylphosphan, 2-(Dicyclohexylphosphan)-biphenyl, 2-(di-tert-butylphosphan)-biphenyl, 2-(Dicyclohexylphosphan)-2'-(N,N-dimethylamino)-biphenyl, Triphenylphosphan, Tris-(o-Tolyl)-phosphan, Natrium 3-(Diphenylphosphino)benzolsulfonat, Tris-2-(Methoxyphenyl)-phosphan, 2,2'-Bis-(diphenylphosphan)-1,1'-binaphthyl, 1,4-Bis-(diphenylphosphan)-butan, 1,2-Bis-(diphenylphosphan)-ethan, 1,4-Bis-(dicyclohexylphosphan)-butan, 1,2-Bis-(dicyclohexylphosphan)-ethan, 2-(Dicyclohexylphosphan)-2'-(N,N-dimethylamino)-biphenyl, Bis(diphenylphosphino)ferrocen oder Tris-(2,4-tert-butylphenyl)-phosphit getrennt zur Reaktion zugibt.
Zur Durchführung des erfindungsgemäßen Verfahrens (b) zur Herstellung der Verbindungen der Formel (I) setzt man pro Mol des Halogen-iodpyrazolylcarboxanilids der Formel (IV) im Allgemeinen 1 bis 15 Mol, vorzugsweise 2 bis 8 Mol an Boronsäurederivat der Formel (V) ein.

Zur Durchführung des erfindungsgemäßen Verfahrens (c) zur Herstellung der Verbindungen der Formel (I) setzt man pro Mol des Iodpyrazolylcarboxamid-Boronsäure-Derivate der Formel (VI) im Allgemeinen 0,8 bis 15 Mol, vorzugsweise 0,8 bis 8 Mol an Phenyl-Derivat der Formel (VII) ein.

Zur Durchführung des erfindungsgemäßen Verfahrens (d) zur Herstellung der Verbindungen der Formel (I) setzt man pro Mol des Halogen-iodpyrazolylcarboxanilides der Formel (IV) im Allgemeinen 0,8 bis 15 Mol, vorzugsweise 0,8 bis 8 Mol an Phenyl-Derivat der Formel (VII) und 0,8 bis 15 Mol, vorzugsweise 0,8 bis 8 Mol an 4,4,4',4',5,5,5',5'-Octamethyl-2,2'-bis-1,3,2-dioxaborolan ein.

Als Verdünnungsmittel zur Durchführung des erfindungsgemäßen Verfahrens (e) kommen alle inerten organischen Lösungsmittel in Betracht. Hierzu gehören vorzugsweise aliphatische oder alicyclische Kohlenwasserstoffe, wie beispielsweise Petrolether, Hexan, Heptan, Cyclohexan, Methylcyclohexan oder Decalin; Ether, wie Diethylether, Diisopropylether, Methyl-t-butylether, Methyl-t-Amylether, Dioxan, Tetrahydrofuran, 1,2- Dimethoxyethan oder 1,2-Diethoxyethan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße (e) Verfahren wird in Gegenwart eines Katalysators durchgeführt. Als solche kommen alle Katalysatoren infrage, die für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Die Hydrierung im erfindungsgemäßen Verfahren (e) kann statt in Gegenwart von Wasserstoff in Kombination mit einem Katalysator auch in Anwesenheit von Triethylsilan durchgeführt werden.

Die Reaktionstemperaturen können bei der Durchführung des erfindungsgemäßen Verfahrens (e) in einem größeren Bereich variiert werden. Im allgemeinen arbeitet man bei Temperaturen von 0°C bis 150°C, vorzugsweise bei Temperaturen von 20°C bis 100°C.

Das erfindungsgemäße Verfahren (e) wird unter einem Wasserstoffdruck zwischen 0.5 and 200 bar, bevorzugt zwischen 2 und 50 bar, besonders bevorzugt zwischen 3 und 10 bar durchgeführt.
Als Verdünnungsmittel zur Durchführung des erfindungsgemäßen Verfahrens (f) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren (f) wird gegebenenfalls in Gegenwart einer Säure durchgeführt. Als solche kommen alle anorganischen und organischen Protonen- wie auch Lewissäuren, sowie auch alle polymeren Säuren infrage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Titantetrachlorid, Tetrabutylorthotitanat, Zinkchlorid, Eisen-III-chlorid, Antimonpentachlorid, saure Ionenaustauscher, saure Tonerden und saures Kieselgel.

Die Reaktionstemperaturen können bei der Durchführung des erfindungsgemäßen Verfahrens (f) in einem größeren Bereich variiert werden. Im allgemeinen arbeitet man bei Temperaturen von 0°C bis 150°C, vorzugsweise bei Temperaturen von 0°C bis 100°C.

Die erfindungsgemäßen Verfahren (f) und (e) können auch in einer Tandemreaktion ("Eintopf-Reaktion") durchgeführt werden. Dazu wird eine Verbindung der Formel (VIII) gegebenenfalls in Gegenwart eines Verdünnungsmittels (geeignete Lösungsmittel wie für Verfahren (f)), gegebenenfalls in Gegenwart einer Säure (geeignete Säuren wie für Verfahren (f)) und in Anwesenheit von Triethylsilan umgesetzt.

Als Verdünnungsmittel zur Durchführung des erfindungsgemäßen Verfahrens (g) kommen alle inerten organischen Lösungsmittel in Betracht. Hierzu gehören vorzugsweise Nitrile, wie Acetonitril, Propionitril, n- oder i-Butyronitril oder Benzonitril oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (g) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Di- methyl-benzylamin, Pyridin, N-Methylpiperidin, N-Methylmopholin, N,N-Dimethylaminopyridin, Diazabicyclooctan (DABCO), Diazabicyclononen (DBN) oder Diazabicycloundecen (DBU).

Das erfindungsgemäße Verfahren (g) wird in Gegenwart eines oder mehrerer Katalysatoren durchgeführt.

Dazu eignen sich besonders Palladiumsalze oder -komplexe. Hierzu kommen vorzugsweise Palladiumchlorid, Palladiumacetat, Tetrakis-(triphenylphosphin)-Palladium oder Bis-(triphenylphosphin)-Palladiumdichlorid infrage. Es kann auch ein Palladiumkomplex in der Reaktionsmischung erzeugt werden, wenn man ein Palladiumsalz und ein Komplexligand getrennt zur Reaktion zugibt.

Als Liganden kommen vorzugsweise Organophosphorverbindungen infrage. Beispielhaft seien genannt: Triphenylphosphin, tri-o-Tolylphosphin, 2,2'-Bis(diphenylphosphino)-1,1'-binaphthyl, Dicyclohexylphosphinebiphenyl, 1,4-Bis(diphenylphosphino)butan, Bisdiphenylphosphinoferrocen, Di(tert.-butylphosphino)biphenyl, Di(cyclohexylphosphino)biphenyl, 2-Dicyclohexylphosphino-2'-N,N-dimethylaminobiphenyl, Tricyclohexylphosphin, Tri-tert.-butylphosphin. Es kann aber auch auf Liganden verzichtet werden.

Das erfindungsgemäße Verfahren (g) wird ferner gegebenenfalls in Gegenwart eines weiteren Metallsalzes, wie Kupfersalzen, beispielsweise Kupfer-(1)-iodid durchgeführt.

Die Reaktionstemperaturen können bei der Durchführung des erfindungsgemäßen Verfahrens (g) in einem größeren Bereich variiert werden. Im allgemeinen arbeitet man bei Temperaturen von 20°C bis 180°C, vorzugsweise bei Temperaturen von 50°C bis 150°C.
Zur Durchführung des erfindungsgemäßen Verfahrens (g) zur Herstellung der Verbindungen der Formel (I) setzt man pro mol des Halogen-iodpyrazolylcarboxanilides der Formel (IV) im Allgemeinen 1 bis 5 mol, vorzugsweise 1 bis 2 mol an Alkin der Formel (IX) oder Alken der Formel (X) ein.

Als Verdünnungsmittel zur Durchführung des erfindungsgemäßen Verfahrens (h) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylfonnanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether.

Das erfindungsgemäße Verfahren (h) wird gegebenenfalls in Gegenwart eines geeigneten Säureakzeptors durchgeführt. Als solche kommen alle üblichen starken Basen infrage. Hierzu gehören vorzugsweise Erdalkalimetall- oder Alkalimetallhydride, -hydroxide, -amide, -alkoholate oder Alkalimetall-Kohlenwasserstoffverbindungen, wie beispielsweise Natriumhydrid, Natriumhydroxid, Kaliumhydroxid, Natriumamid, Lithiumdiisopropylamid, Natriummethylat, Natriumethylat, Kalium-tert.-butylat, Methyllithium, Phenyllithium oder Butyllithium.

Die Reaktionstemperaturen können bei der Durchführung des erfindungsgemäßen Verfahrens (h) in einem größeren Bereich variiert werden. Im allgemeinen arbeitet man bei Temperaturen von -80°C bis 150°C, vorzugsweise bei Temperaturen von -30°C bis 80°C.

Zur Durchführung des erfindungsgemäßen Verfahrens (h) zur Herstellung der Verbindungen der Formel (I) setzt man pro mol des Ketons der Formel (XI) im Allgemeinen 1 bis 5 mol, vorzugsweise 1 bis 2 mol an Phosphorverbindung der Formel (XII) ein.

Als Verdünnungsmittel zur Durchführung des erfindungsgemäßen Verfahrens (i) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2- Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (i) wird in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (i) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens (i) zur Herstellung der Verbindungen der Formel (I) setzt man pro Mol des Iodpyrazolylcarboxanilids der Formel (Ib) im Allgemeinen 0,2 bis 5 Mol, vorzugsweise 0,5 bis 2 Mol an Halogenid der Formel (XIII) ein.

Alle erfindungsgemäßen Verfahren werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen. Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella hepotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Puccinia-Arten und von Krankheiten im Wein-, Obst- und Gemüseanbau, wie beispielsweise gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfingdungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium glöbosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Füllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

2-Phenylphenol; 8-Hydroxychinolinsulfat; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamin; Calcium polysulfide; Capsimycin; Captafol; Captan; Carbendazim; Carboxin; Carpropamid; Car-vone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2-propinyl]oxy]-3-methoxyphenyl]ethyl]-3-methyl-2-[(methylsulfonyl)amino]-butanamid; 1-(1-Naphthalenyl)-1H-pyrrol-2,5-dion; 2,3,5,6-Tetrachlor-4-(methylsulfonyl)-pyridin; 2-Amino-4-methyl-N-phenyl-5-thiazolcarboxamid; 2-Chlor-N-(2,3-dihydro-1,1,3-trimethyl-1H-inden-4-yl)-3-pyridincarboxamide; 3,4,5-Trichlor-2,6-pyridindicarbonitril; Actinovate; cis-1-(4-Chlorphenyl)-2-(1H-1,2,4-triazol-1-yl)-cycloheptanol; Methyl 1-(2,3-dihydro-2,2-dimethyl-1H-inden-1-yl)-1H-imidazol-5-carboxylat; Monokaliumcarbonat; N-(6-Methoxy-3-pyridinyl)-cyclopropancarboxamid; N-Butyl-8-(1,1-dimethylethyl)-1-oxaspiro[4.5]decan-3-amin; Natriumtetrathiocarbonat; sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avennectin, AZ-60541, Azadirachtin, Azamethiphos, Azinphos-methyl, Azinphos-ethyl, Azocyclotin, Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben, Cadusafos, Camphechlor, Carbaryl, Carbofuran, Carbophenothion, Carbosulfan, Cartap, CGA-50439, Chinomethionat, Chlordane, Chlordime-form, Chloethocarb, Chlorethoxyfos, Chlorfenapyr, Chlorfenvinphos, Chlorfluazuron, Chlormephos, Chlorobenzilate, Chloropicrin, Chlorproxyfen, Chlopyrifos-methyl, Chlorpyrifos (-ethyl), Chlovaporthrin, Chromafenozide, Cis-Cypennethrin, Cis-Resmethrin, Cis-Perrnetl rin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (IR-trans-isomer), Cyromazine, DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439, Eflusilanate, Emamectin, Emamectin-benzoate, Empenthrin (IR-isomer), Endosulfan, Entomopthora spp., EPN, Esfenvalerate, Ethiofencarb, Ethiprole, Ethion, Ethoprophos, Etofenprox, Etoxazole, Etrimfos, Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb, Gamma-HCH, Gossyplure, Grandlure, Granuloseviren, Halfenprox, Halofenozide, HCH, HCN-801, Heptenophos, Hexaflumuron, Hexythiazox, Hydramethylnone, Hydroprene, IKA-2002, Imidacloprid, Imiprothrin, Indoxacarb, Iodofenphos, Iprobenfos, Isazofos, Isofenphos, Isoprocarb, Isoxathion, Ivermectin, Japonilure, Kadethrin, Kernpolyederviren, Kinoprene, Lambda-Cyhalothrin, Lindane, Lufenuron, Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800, Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron, OK-5101, OK-5201, OK-9601, OK-9602, OK-9701, OK-9802, Omethoate, Oxamyl, Oxydemeton-methyl, Paecilomyces fumosoroseus, Parathion-methyl, Parathion (-ethyl), Permethrin (cis-, trans-), Petroleum, PH-6045, Phenothrin (IR-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen, Quinalphos, Resmethrin, RH-5849, Ribavirin, RU-12457, RU-15525, S-421, S-1833, Salithion, Sebufos, SI-0009, Silafluofen, Spinosad, Spirodiclofen, Spiromesifen, Sulfluramid, Sulfotep, Sulprofos, SZI-121, Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (IR-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb, Vamidothion, Vaniliprole, Verbutin, Verticillium lecanii, WL-108477, WL-40027, YI-5201, YI-5301, YI-5302, XMC, Xylylcarb, ZA-3274, Zeta-Cypermethrin, Zolaprofos, ZXI-8901, die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im Allgemeinen zwischen 0,1 und 10.000 g/ha, vorzugsweise zwischen 10 und 1.000 g/ha. Bei der Saatgutbehandlung liegen die Aufwandmengen an Wirkstoff im Allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Hersteilungsbeispiele

### Beispiel 1

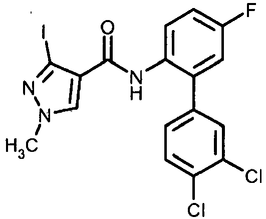

273 mg (1,0 mmol) 3-Iod-1-methyl-1*H*-pyrazol-4-carbonsäure, 213 mg (0,83 mmol) 3',4'-Dichlor-5-fluor-biphenyl-2-amin, 0,3 ml (1,67 mmol) N,N-Diisopropylethylamin und 583 mg (1,25 mmol) Brom-tripyrrolidinophosphonium-hexafluorophosphat wurden in 5 ml Methylenchlorid 3 Tage bei Raumtemperatur gerührt. Das Gemisch wurde mit gesättigter Natriumhydrogencarbonat-Lösung und anschließend mit Wasser gewaschen. Nach Abtrennen und Einengen der organischen Phase erhielt man 910 mg Rohprodukt. Nach säulenchromatographischer Reinigung über Kieselgel 60 mit Methylenchlorid/Diethylether 5:1 wurden 230 mg (53,5 % der Theorie) an *N*-(3',4'-Dichlor-5-fluor-1,1'-biphenyl-2-yl)-3-iod-1-methyl-1*H*-pyrazol-4-carboxamid (Verbindung Nr. 6 aus Tabelle 1) mit dem logP (pH 2,3) = 3.42 erhalten.

Analog Beispiel 1, sowie entsprechend den Angaben in der allgemeinen Beschreibung der erfindungsgemäßen Herstellverfahren (a) bis (i) wurden auch die in der nachstehenden Tabelle 1 genannten Verbindungen der Formel (I) erhalten:

**Tabelle 1**

| | | | | | | | | |
|---|---|---|---|---|---|---|---|---|
| 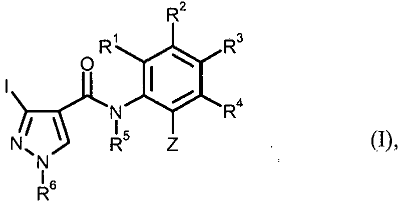 | | | | | | | | |

| **Nr.** | **Z** | **R⁴** | **R⁶** | **R⁵** | **R³** | **R²** | **R¹** | **logP** |
|---|---|---|---|---|---|---|---|---|
| 2 | 3,4-Dichlophenyl | H | CH₃ | H | H | H | H | 3.38 |
| 3 | 4-Chlor-3-fluophenyl | H | CH₃ | H | H | H | H | 3.09 |
| 5 | 3-Fluor-4-propoxyiminomethyl-phenyl | H | CH₃ | H | H | H | H | 4.00 |
| 6 | 3,4-Dichlorphenyl | H | CH₃ | H | F | H | H | 3.42 |
| 7 | 3-Fluor-4-methylphenyl | H | CH₃ | H | H | H | H | 3.18 |
| 8 | 3-Chlor-4-fluophenyl | H | CH₃ | H | H | H | H | 3.09 |
| 9 | 4-Bromphenyl | H | CH₃ | H | H | H | H | 3.20 |
| 10 | 4- Trifluormethylphenyl | H | CH₃ | H | H | H | H | 3.24 |
| 11 | 3-Fluor-4-trifluormethylphenyl | H | CH₃ | H | H | H | H | 3.23 |
| 14 | 4-Chlorphenyl | H | CH₃ | H | H | H | H | 3.08 |
| 16 | 4-Iodphenyl | H | CH₃ | H | H | H | H | 3.36 |
| 17 | 3-Chlor-2-fluorphenyl | H | CH₃ | H | H | H | H | 2.99 |
| 23 | 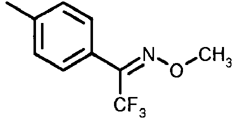 | H | CH₃ | H | H | H | H | 3,70 |
| 28 | 2,3-Dichlorphenyl | H | CH₃ | H | H | H | H | 3,34 |
| 29 | 4-Chlor-3 -fluorphenyl | H | CH₃ | H | H | H | F | 2,78 |
| 30 | 2-Chlor-4-trifluormethylphenyl | H | CH₃ | H | H | H | H | 3,55 |
| 31 | 4-Brom-3-chlorphenyl | H | CH₃ | H | H | H | H | 3,45 |
| 32 | 4-Brom-2-chlorphenyl | H | CH₃ | H | H | H | H | 3,56 |

### Herstellung der Ausganasstoffe der Formel (II)

### Beispiel (II-1)

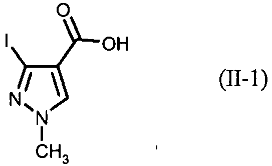

### Schritt 1:

13 ml Isoamylnitrit wurden in 80 ml Methyleniodid vorgelegt. Bei 100°C wurden 10.2 g (60.3 mmol) 3-Amino-1-methyl-1*H*-pyrazol-4-carbonsäure-ethylester (XIV-1) zugetropft. Nach 15 min Rühren bei dieser Temperatur wurde eingeengt.
Man erhielt 15.2 g (79 % der Theorie) an 3-Iod-1-methyl-1*H*-pyrazol-4-carbonsäure-ethylester [logP (pH 2.3) = 1.74], welcher ohne weitere Aufarbeitung weiter eingesetzt wurde.

### Schritt 2:

134 g (0.478 mol) 3-Iod-1-methyl-1*H*-pyrazol-4-carbonsäure-ethylester wurden in 850 ml Ethanol vorgelegt und eine Lösung von 29.5 g (0.526 mol) KOH in 340 ml Wasser zugetropft. Nach 2 Tagen Rühren bei Raumtemperatur wurde eingeengt, der Rückstand in Wasser aufgenornrnen, mit Essigsäureethylester extrahiert und nach Abtrennung die wässrige Phase mit Salzsäure auf pH 1 eingestellt, wobei ein Feststoff ausfiel. Absaugen und 3 Stunden Trocknen im Vakuum bei 40°C lieferte 88 g (70 % der Theorie) an 3-Iod-1-methyl-1*H*-pyrazol-4-carbonsäure [logP pH 2.3) = 0.57]. Die wässrige Phase wurde mit Essigsäureethylester extrahiert und die organische Phase eingeengt, was noch mal 5.1 g (2.1 % der Theorie) des Produktes lieferte.

### Herstellung der Ausgangsstoffe der Formel (XIV)

### Beispiel (XIV-1)

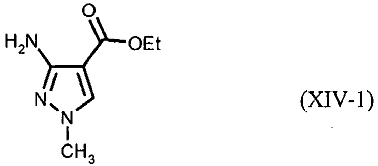

In eine Suspension von 220 g (0.855 mol) 3-(*N*'-Benzyliden-*N*-methyl-hydrazino)-2-cyano-acrylsäure-ethylester (XVI-1) in 1000 ml Ethanol wurden bei Raumtemperatur innerhalb von 20 min 100 ml konzentrierte Salzsäure zugetropft. Anschließend wurde 1 Stunde zum Sieden erhitzt. Nach Entfernung des Lösemittels wurde der ölige Rückstand unter leichtem Erwärmen mit 200 ml Diethylether verrührt, wobei ein Feststoff ausfiel. Absaugen lieferte 149 g (95 % der Theorie) an 3-Amino-1-methyl-1*H*-pyrazol-4-carbonsäure-ethylester [logP (pH 2.3) = 0.72].

### Herstellung der Ausgangsstoffe der Formel (XVI)

### Beispiel (XVI-1)

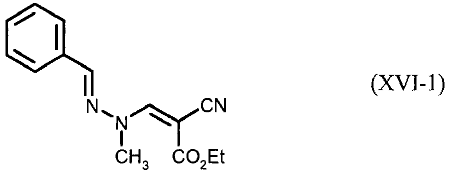

258g (1.92 mol) *N*-Benzyliden-*N'*-methyl-hydrazin und 325 g (1.92 mol) Ethoxymethylencyanessigsäureethylester wurden in 1000 ml Toluol vorgelegt und 1 Stunde zum Sieden erhitzt. Nach dem Abkühlen wurde abgesaugt, was 447 g (89,5 % der Theorie) an 3-(*N*'-Benzyliden-*N*-methyl-hydrazino)-2-cyano-acrylsäure-ethylester [logP (pH 2.3) = 2.31] lieferte. Das Filtrat wurde für 16 Stunden stehen gelassen und erneut abgesaugt, was noch mal 7.7 g (1.5 % der Theorie) des gewünschten Produktes lieferte.

Die Bestimmung der angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
Eluenten für die Bestimmung im sauren Bereich (pH 2,3): 0,1 % wässrige Phosphorsäure, Acetonitril; linearer Gradient von 10 % Acetonitril bis 90 % Acetonitril.
Die Eichung erfolgte mit unverzweigten Alkan-2-onen (mit 3 bis 16 Kohlenstoffatomen), deren LogP-Werte bekannt sind (Bestimmung der LogP-Werte anhand der Retentionszeiten durch lineare Interpolation zwischen zwei aufeinanderfolgenden Alkanonen).
Die lambda-max-Werte wurden an Hand der UV-Spektren von 200 nm bis 400 nm in den Maxima der chromatographischen Signale ermittelt.

### Anwendungsbeispiele

### Beispiel A

### Podosphaera-Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers *Podosphaera leucotricha* inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse sind der folgenden Tabelle zu entnehmen.

**Tabelle A**

| **Podosphaera-Test (Apfel) / protektiv** | | |
|---|---|---|
| Wirkstoff Erfindungsgemäß | Aufwandmenge an Wirkstoff in g/ha | Wirkungsgrad in % |
| 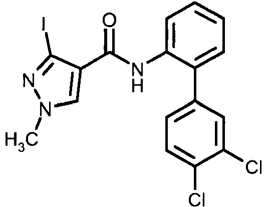 | 100 | 99 |
| 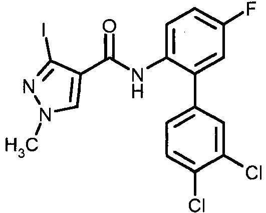 | 100 | 100 |
| 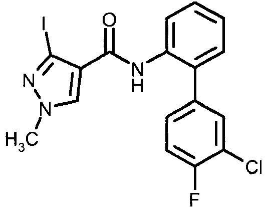 | 100 | 100 |
| 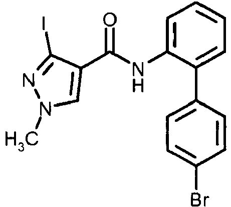 | 100 | 100 |
| 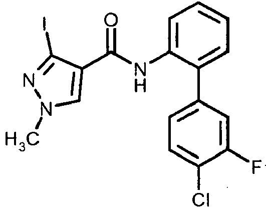 | 100 | 100 |
| 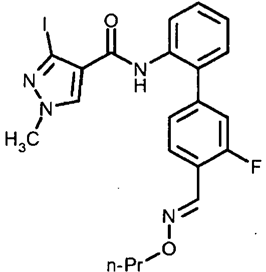 | 100 | 100 |
| 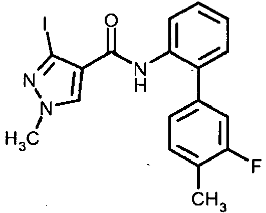 | 100 | 100 |
| 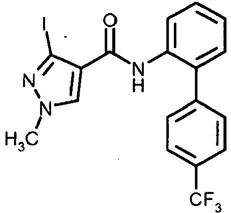 | 100 | 88 |
| 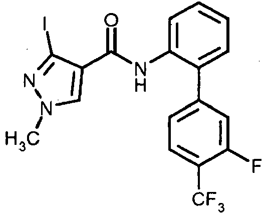 | 100 | 99 |
| 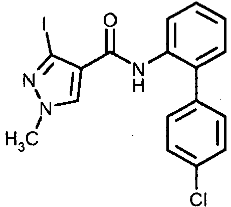 | 100 | 100 |
| 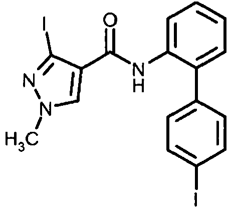 | 100 | 100 |
| 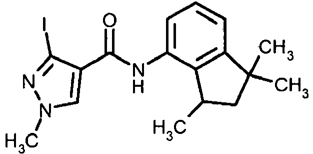 | 100 | 100 |

### Beispiel B

### Venturia - Test (Apfel) / protektiv

| | | |
|---|---|---|
| Lösungsmittel: | 24,5 | Gewichststeile Aceton |
| | 24,5 | Gewichtsteile Dimethylacetamid |
| Emulgator : | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse sind der folgenden Tabelle zu entnehmen.

**Tabelle B**

| **Venturia - Test (Apfel) / protektiv** | | |
|---|---|---|
| Wirkstoff Erfindungsgemäß | Aufwandmenge an Wirkstoff in g/ha | Wirkungsgrad in % |
| 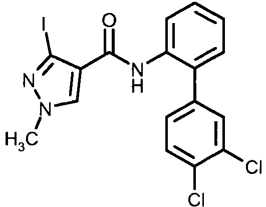 | 100 | 99 |
| 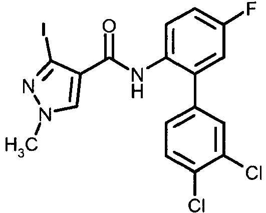 | 100 | 100 |
| 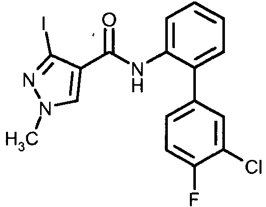 | 100 | 100 |
| 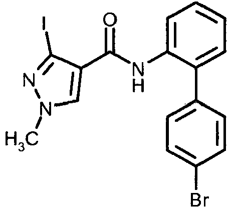 | 100 | 100 |
| 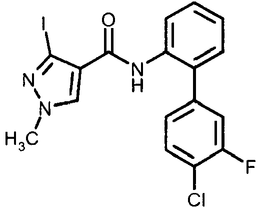 | 100 | 100 |
| 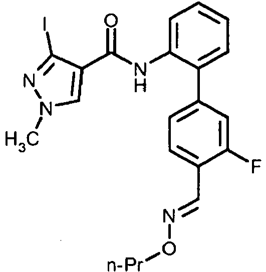 | 100 | 100 |
| 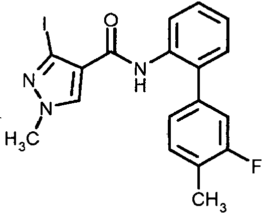 | 100 | 97 |
| 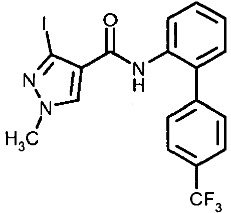 | 100 | 94 |
| 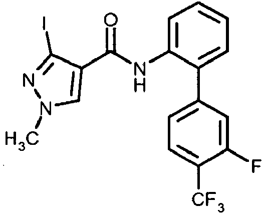 | 100 | 98 |
| 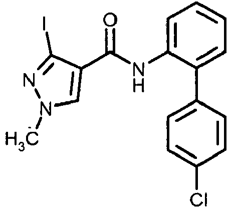 | 100 | 100 |
| 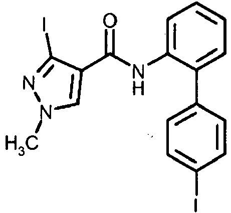 | 100 | 99 |
| 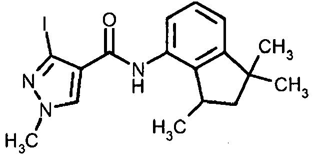 | 100 | 100 |

### Beispiel C

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinereca* bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse sind der folgenden Tabelle zu entnehmen.

**Tabelle C**

| **Botrytis - Test (Bohne) / protektiv** | | |
|---|---|---|
| Wirkstoff Erfindungsgemäß | Aufwandmenge an Wirkstoff in g/ha | Wirkungsgrad in % |
| 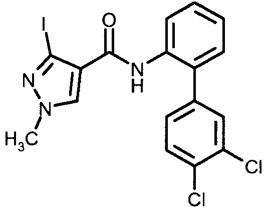 | 500 | 100 |
| 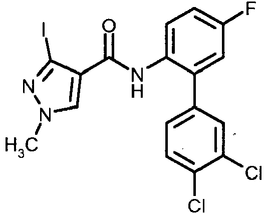 | 100 | 100 |
| 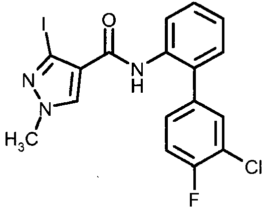 | 500 | 100 |
| 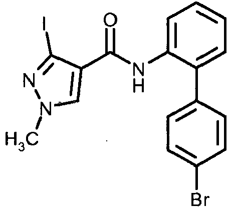 | 500 | 100 |
| 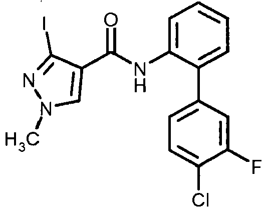 | 500 | 100 |
| 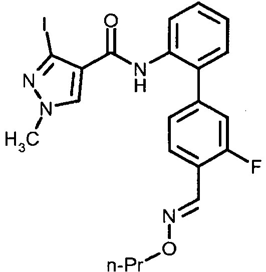 | 500 | 100 |
| 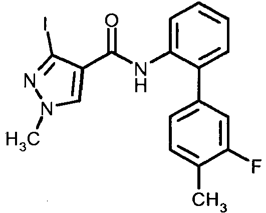 | 500 | 100 |
| 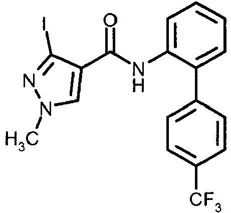 | 500 | 94 |
| 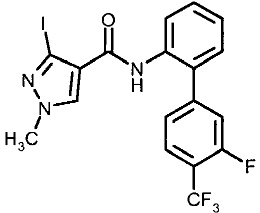 | 500 | 100 |
| 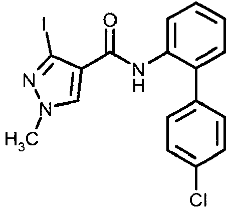 | 100 | 100 |
| 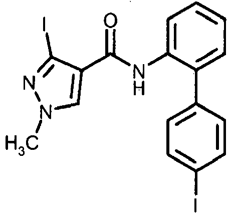 | 100 | 100 |
| 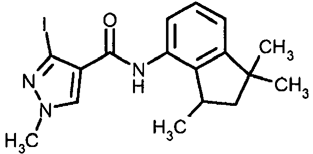 | 100 | 100 |

### Beispiel D

### Pyrenophora teres-Test (Gerste) / protektiv

| | | |
|---|---|---|
| Lösungsmittel: | 50 | Gewichtsteile *N*,*N*-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegeben Aufwandmenge besprüht. Nach Antrocknen des Spritzbelages werden die Pflanzen mit einer Konidiensuspension von *Pyrenophora teres* besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und relativen Luftfeuchtigkeit von ca. 80 % aufgestellt.

8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse sind der folgenden Tabelle zu entnehmen.

**Tabelle D**

| **Pyrenophora teres-Test (Gerste) / protektiv** | | |
|---|---|---|
| Wirkstoff Erfindungsgemäß | Aufwandmenge an Wirkstoff in g/ha | Wirkungsgrad in % |
| 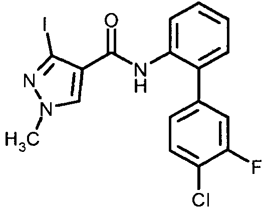 | 500 | 100 |
| 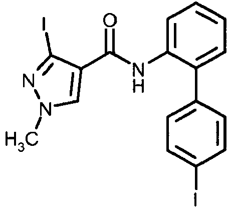 | 500 | 100 |
| 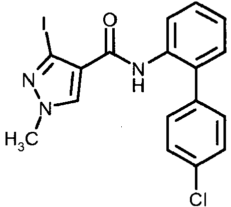 | 500 | 100 |
| 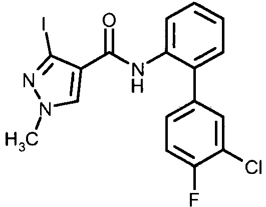 | 500 | 100 |
| 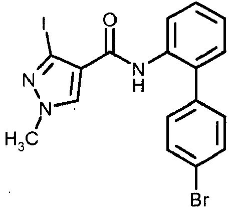 | 500 | 100 |
| 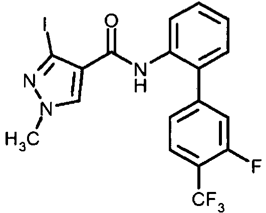 | 500 | 100 |

### Beispiel E

### Puccinia-Test (Weizen) / protektiv

| | | |
|---|---|---|
| Lösungsmittel: | 50 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von *Puccinia recondita* besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse sind der folgenden Tabelle zu entnehmen.

**Tabelle E**

| **Puccinia-Test (Weizen) / protektiv** | | |
|---|---|---|
| Wirkstoff Erfindungsgemäß | Aufwandmenge an Wirkstoff in g/ha | Wirkungsgrad in % |
| 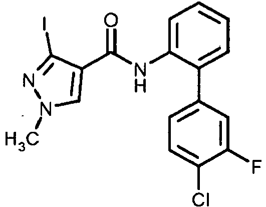 | 500 | 100 |
| 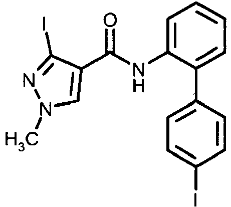 | 500 | 100 |
| 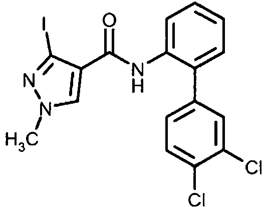 | 500 | 100 |
| 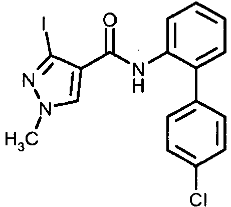 | 500 | 100 |
| 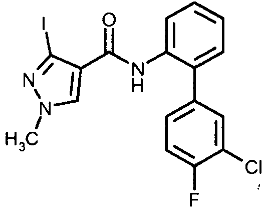 | 500 | 100 |
| 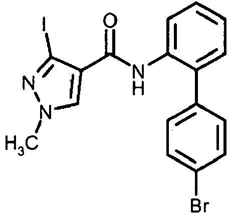 | 500 | 100 |
| 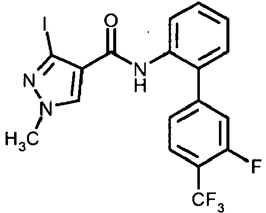 | 500 | 100 |
| 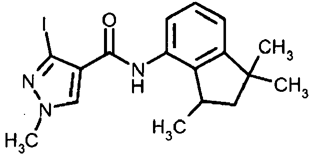 | 500 - | 100 |

### Beispiel F

### Alternaria-Test (Tomate) / protektiv

| | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von *Alternaria solani* inokuliert und stehen dann 24 h bei 100 % relativer Luftfeuchtigkeit und 20°C. Anschließend stehen die Pflanzen bei 96 % relativer Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse sind der folgenden Tabelle zu entnehmen.

**Tabelle F**

| **Alternaria-Test (Tomate) / protektiv** | | |
|---|---|---|
| Wirkstoff Erfindungsgemäß | Aufwandmenge an Wirkstoff in g/ha | Wirkungsgrad in % |
| 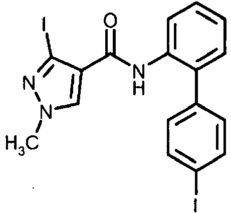 | 750 | 100 |
| 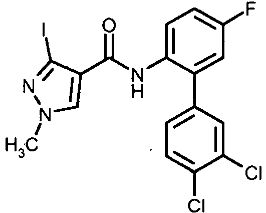 | 750 | 100 |
| 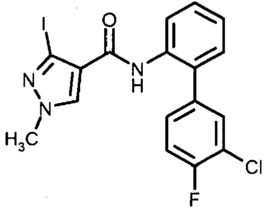 | 750 | 100 |

## Patentansprüche

1. Iodpyrazolylcarboxanilide der Formel (I) 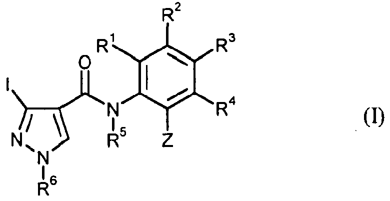 in welcher
R¹, R², R³ und R⁴ unabhängig voneinander für Wasserstoff, Fluor, Chlor, Methyl, iso-Propyl oder Methylthio stehen,
R⁵ für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/ oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁷, -CONR⁸R⁹ oder -CH₂NR¹⁰R¹¹ steht,
R⁶ für C₁-C₃-Alkyl, C₁-C₂-Alkoxy-C₁-C₂-alkyl, C₁-C₃-Halogenalkyl mit 1 bis 7 Fluor-, Chlor- und/oder Bromatomen steht,
R⁷ für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen, -COR¹² steht,
R⁸ und R⁹ unabhängig voneinander für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
R⁸ und R⁹ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹³ enthalten kann,
R¹⁰ und R¹¹ unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
R¹⁰ und R¹¹ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹³ enthalten kann,
R¹² für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
R¹³ für Wasserstoff oder C₁-C₆-Alkyl steht,
Z für Z¹, Z² oder Z³ steht, worin
Z¹ für gegebenenfalls einfach bis fünffach, gleich oder verschieden substituiertes Phenyl steht,
oder
R¹, R² und R³ unabhängig voneinander für Wasserstoff, Fluor oder Chlor stehen und
Z und R⁴ gemeinsam mit den Kohlenstoffatomen, an die sie gebunden sind, einen gegebenenfalls substituierten 5- oder 6-gliedrigen carbocyclischen oder heterocyclischen Ring bilden.

2. Iodpyrazolylcarboxanilide der Formel (**I**) gemäß Anspruch 1, in welcher
R¹, R², R³ und R⁴ unabhängig voneinander für Wasserstoff, Fluor, Chlor oder Methyl stehen,
R⁵ für Wasserstoff; C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₆-Halogencycloalkyl mit jeweils 1 bis 9 Fluor-, Chlor- und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁷, -CONR⁸R⁹ oder -CH₂NR¹⁰R¹¹ steht,
R⁶ für Methyl, Ethyl, iso-Propyl, Monofluormethyl, Difluormethyl oder Trifluormethyl steht,
R⁷ für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄- Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen, -COR¹² steht,
R⁸ und R⁹ unabhängig voneinander für Wasserstoff, C₁-C₆-Alkyl, C₁-C₃-Alkoxy-C₁-C₃-alkyl, C₃-C₆-Cycloalkyl; C₁-C₄-Halogenalkyl, Halogen-C₁-C₃-alkoxy-C₁-C₃-alkyl, C₃-C₆-Halogencycloalkyl mit jeweils 1 bis 9 Fluor-, Chlor- und/oder Bromatomen stehen,
R¹⁰ und R¹¹ unabhängig voneinander für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor- und/oder Bromatomen stehen,
R¹⁰ und R¹¹ außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹³ enthalten kann,
R¹² für Wasserstoff, C₁-C₆-Alkyl, C₁-C₄-Alkoxy, C₁-C₃-Alkoxy-C₁-C₃-alkyl, C₃-C₆-Cycloalkyl; C₁-C₄-Halogenalkyl, C₁-C₄-Halogenalkoxy, Halogen-C₁-C₃-alkoxy-C₁-C₃-alkyl, C₃-C₆-Halogencycloalkyl mit jeweils 1 bis 9 Fluor-, Chlor- und/oder Bromatomen steht,
R¹³ für Wasserstoff oder C₁-C₄-Alkyl steht,
Z für Z¹, Z² oder Z³ steht, worin
Z¹ für gegebenenfalls einfach bis fünffach, gleich oder verschieden substituiertes Phenyl steht, wobei die Substituenten aus der Liste W¹ ausgewählt sind,
W¹ für Halogen, Cyano, Nitro, Amino, Hydroxy, Formyl, Carboxyl, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy mit 1 bis 6 Kohlenstoffatomen in den jeweiligen Kohlenwasserstoffketten, Alkenylcarbonyl oder Alkinylcarbonyl, mit 2 bis 6 Kohlenstoffatomen in den jeweiligen Kohlenwasserstofflcetten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder eine Gruppierung -C(Q¹)=N-Q² steht, worin
Q¹ für Wasserstoff, Hydroxy, Alkyl mit 1 bis 4 Kohlenstoffatomen, Halogenalkyl mit 1 bis 4 Kohlenstoffatomen und 1 bis 9 gleichen oder verschiedenen Halogenatomen oder Cycloalkyl mit 1 bis 6 Kohlenstoffatomen steht und
Q² für Hydroxy, Amino, Methylamino, Phenyl, Benzyl oder für jeweils gegebenenfalls durch Halogen, Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht,
sowie für jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen steht,
Z und R⁴ auch gemeinsam mit den Kohlenstoffatomen, an die sie gebunden sind, für einen gegebenenfalls einfach bis vierfach, gleich oder verschieden substituierten 5- oder 6-gliedrigen carbocyclischen oder heterocyclischen Ring stehen.

3. Verfahren zum Herstellen der Iodpyrazolylcarboxanilide der Formel (I) gemäß Anspruch 1, **dadurch gekennzeichnet, dass** man
a) Iodpyrazolylcarbonsäurederivate der Formel (II) 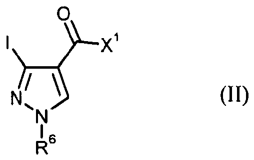 in welcher
R⁶ die in Anspruch 1 angegebenen Bedeutungen hat und
X¹ für Chlor oder Hydroxy steht,
mit Anilin-Derivaten der Formel (III) 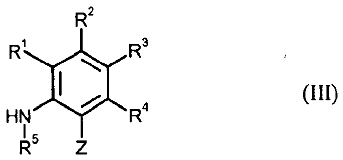 in welcher R¹, R², R³, R⁴, R⁵ und Z die in Anspruch 1 angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt,
oder
b) Halogen-iodpyrazolylcarboxanilide der Formel (IV) 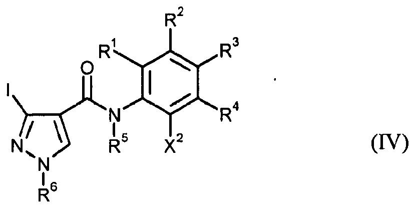 in welcher
R¹, R², R³, R⁴, R⁵ und R⁶ die in Anspruch 1 angegebenen Bedeutungen haben, und
X² für Chlor, Brom, Iod oder Trifluormethylsulfonat steht,
mit Boronsäure-Derivaten der Formel (V) 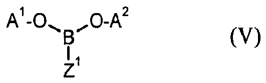 in welcher
Z¹ die in Anspruch 1 angegebene Bedeutung hat und
A¹ und A² jeweils für Wasserstoff oder zusammen für Tetramethylethylen stehen, in Gegenwart eines Katalysators, gegebenenfalls in Gegenwart eines Säurebindemittels und gegebenenfalls in Gegenwart eines Verdünnungsmittels umsetzt,
oder
c) Iodpyrazolylcarboxamid-Boronsäure-Derivate der Formel (VI) 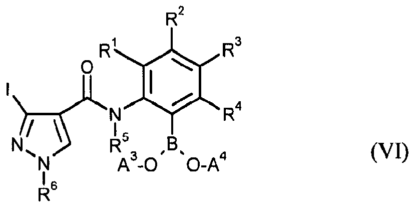 in welcher
R¹, R², R³, R⁴, R⁵ und R⁶ die in Anspruch 1 angegebenen Bedeutungen haben, und
A³ und A⁴ jeweils für Wasserstoff oder zusammen für Tetramethylethylen stehen,
mit Phenyl-Derivaten der Formel (VII)
X³-Z¹ (VII)
in welcher
Z¹ die in Anspruch 1 angegebenen Bedeutungen hat und
X³ für Chlor, Brom, Iod oder Trifluormethylsulfonat steht,
in Gegenwart eines Katalysators, gegebenenfalls in Gegenwart eines Säurebindemittels und gegebenenfalls in Gegenwart eines Verdünnungsmittels umsetzt,
oder
d) Halogen-iodpyrazolylcarboxanilide der Formel (IV) 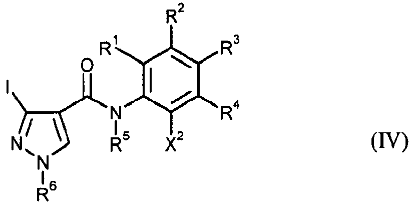 in welcher
R¹, R², R³, R⁴, R⁵ und R⁶ die in Anspruch 1 angegebenen Bedeutungen haben, und
X² für Chlor, Brom, Iod oder Trifluormethylsulfonat steht,
mit Phenyl-Derivaten der Formel (VII) X³-Z¹ (VII) in welcher
Z¹ die in Anspruch 1 angegebenen Bedeutungen hat und
X³ für Chlor, Brom, Iod oder Trifluormethylsulfonat steht,
in Gegenwart eines Palladium- oder Nickel-Katalysators und in Gegenwart von 4,4,4',4',5,5,5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt,
oder
e) Iodpyrazolylcarboxanilide der Formel (Ia) 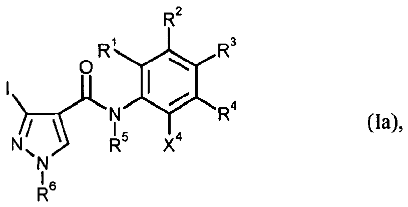 in welcher
R¹, R², R³, R⁴, R⁵ und R⁶ die in Anspruch 1 angegebenen Bedeutungen haben und
X⁴ für jeweils gegebenenfalls einfach oder mehrfach, gleich oder verschieden durch Halogen und/oder C₃-C₆-Cycloalkyl substituiertes C₂-C₂₀-Alkenyl oder C₂-C₂₀-Alkinyl steht, wobei der Cycloalkylteil seinerseits gegebenenfalls durch Halogen und/oder C₁-C₄-Alkyl substituiert sein kann,
gegebenenfalls in Gegenwart eines Verdünnungsmittels und gegebenenfalls in Gegenwart eines Katalysators hydriert,
oder
f) Hydroxyalkyl-iodpyrazolylcarboxanilide der Formel (VIII) 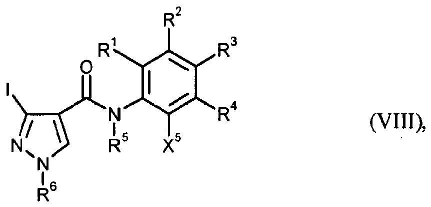 in welcher
R¹, R², R³, R⁴, R⁵ und R⁶ die in Anspruch 1 angegebenen Bedeutungen haben und
X⁵ für gegebenenfalls zusätzlich einfach oder mehrfach, gleich oder verschieden durch Halogen und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann,
gegebenenfalls in Gegenwart eines Verdünnungsmittels und gegebenenfalls in Gegenwart einer Säure dehydratisiert,
oder
g) Halogen-iodpyrazolylcarboxanilide der Formel (IV) 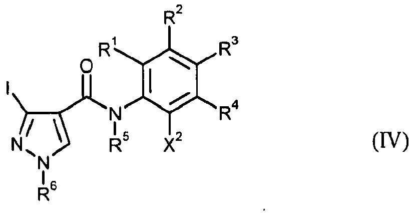 in welcher
R¹, R², R³, R⁴, R⁵ und R⁶ die in Anspruch 1 angegebenen Bedeutungen haben, und
X² für Chlor, Brom, Iod oder Trifluormethylsulfonat steht,
mit einem Alkin der Formel (IX) 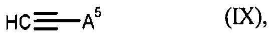 in welcher
A⁵ für jeweils gegebenenfalls einfach oder mehrfach, gleich oder verschieden durch Halogen und/oder C₃-C₆-Cycloalkyl substituiertes C₂-C₁₈-Alkyl steht, wobei der Cycloalkylteil seinerseits gegebenenfalls durch Halogen und/oder C₁-C₄-Alkyl substituiert sein kann,
oder einem Alken der Formel (X) 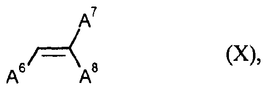 in welcher
A⁶, A⁷ und A⁸ unabhängig voneinander jeweils für Wasserstoff oder gegebenenfalls einfach oder mehrfach, gleich oder verschieden durch Halogen und/ oder C₃-C₆-Cycloalkyl substituiertes Alkyl stehen, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils die Zahl 20 nicht übersteigt,
gegebenenfalls in Gegenwart eines Verdünnungsmittels, gegebenenfalls in Gegenwart eines Säurebindemittels und in Gegenwart eines oder mehrerer Katalysatoren umsetzt,
oder
h) Ketone der Formel (XI) 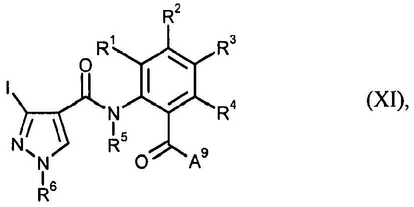 in welcher
R¹, R², R³, R⁴, R⁵ und R⁶ die in Anspruch 1 angegebenen Bedeutungen haben und
A⁹ für Wasserstoff oder gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
mit einer Phosphorverbindung der allgemeinen Formel (XII)
A¹⁰-Px (XII),
in welcher
A¹⁰ für gegebenenfalls einfach oder mehrfach, gleich oder verschieden durch Halogen und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
Px für eine Gruppierung -P⁺ (C₆H₅)₃ Cl⁻, -P⁺ (C₆H₅)₃ Br⁻, -P⁺ (C₆H₅)₃ I⁻, -P(=O)(OCH₃)₃ oder -P(=O)(OC₂H₅)₃ steht,
gegebenenfalls in Gegenwart eines Verdünnungsmittels umsetzt, oder
i) Iodpyrazolylcarboxanilide der Formel (Ib) 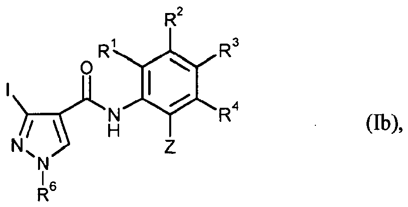 in welcher
R¹, R², R³, R⁴, R⁶ und Z die in Anspruch 1 angegebenen Bedeutungen haben
mit einem Halogenid der Formel (XIII)
R⁵⁻¹-X⁶ (XIII)
in welcher
R⁵⁻¹ für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor-und/oder Bromatomen, Formyl-C₁-C₃-alkyl, (C₁-C₃-Alkyl)carbonyl-C₁-C₃-alkyl, (C₁-C₃-Alkoxy)carbonyl-C₁-C₃-alkyl; (C₁-C₃-Halogenalkyl)carbonyl-C₁-C₃-alkyl, (C₁-C₃-Halogenalkoxy)carbonyl-C₁-C₃-alkyl mit jeweils 1 bis 7 Fluor-, Chlor- und/ oder Bromatomen, (C₁-C₃-Alkyl)carbonyl-C₁-C₃-halogenalkyl, (C₁-C₃-Alkoxy)carbonyl-C₁-C₃-halogenalkyl mit jeweils 1 bis 6 Fluor-, Chlor- und/oder Bromatomen, (C₁-C₃-Halogenalkyl)carbonyl-C₁-C₃-halogenalkyl, (C₁-C₃-Halogenalkoxy)carbonyl-C₁-C₃-halogenalkyl mit jeweils 1 bis 13 Fluor-, Chlor- und/oder Bromatomen; -COR⁷, -CONR⁸R⁹ oder -CH₂NR¹⁰R¹¹ steht,
R⁷, R⁸, R⁹, R¹⁰ und R¹¹ die in Anspruch 1 angegebenen Bedeutungen haben und
X⁶ für Chlor, Brom oder Iod steht,
in Gegenwart einer Base und in Gegenwart eines Verdünnungsmittels umsetzt.

4. Mittel zur Bekämpfung unerwünschter Mikroorganismen, **gekennzeichnet durch** einen Gehalt an mindestens einem Iodpyrazolylcarboxanilid der Formel (I) gemäß Anspruch 1 neben Streckmitteln und/oder oberflächenaktiven Stoffen.

5. Verwendung von lodpyrazolylcarboxaniliden der Formel (I) gemäß Anspruch 1 zur Bekämpfung unerwünschter Mikroorganismen.

6. Verfahren zur Bekämpfung unerwünschter Mikroorganismen, **dadurch gekennzeichnet, dass** man Iodpyrazolylcarboxanilide der Formel (I) gemäß Anspruch 1 auf die Mikroorganismen und/oder deren Lebensraum ausbringt.

7. Verfahren zur Herstellung von Mitteln zur Bekämpfung unerwünschter Mikroorganismen, **dadurch gekennzeichnet, dass** man Iodpyrazolylcarboxanilide der Formel (I) gemäß Anspruch 1 mit Streckmitteln und/oder oberflächenaktiven Stoffen vermischt.

8. Hydroxyalkyl-iodpyrazolylcarboxanilide der Formel (VIII) 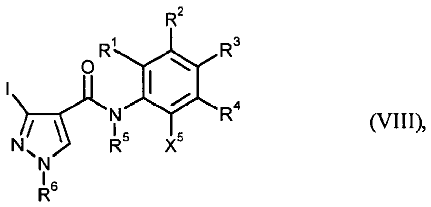 in welcher
R¹, R², R³, R⁴, R⁵ und R⁶ die in Anspruch 1 angegebenen Bedeutungen haben,
X⁵ für gegebenenfalls zusätzlich einfach oder mehrfach, gleich oder verschieden durch Halogen und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann.

9. Iodpyrazolylcarbonsäurederivate der Formel (II) 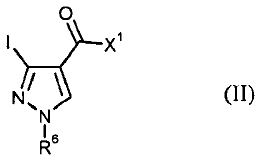 in welcher
R⁶ die in Anspruch 1 angegebenen Bedeutungen hat und
X¹ für Chlor oder Hydroxy steht.

10. Verfahren zum Herstellen von lodpyrazolylcarbonsäurederivate der Formel (II) gemäß Anspruch 9, **dadurch gekennzeichnet, dass** man
j) 3-Amino-pyrazol-4-carbonsäureester der Formel (XIV) 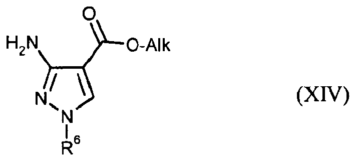 in welcher
Alk für C₁-C₄-Alkyl steht und
R⁶ die in Anspruch 1 angegebenen Bedeutungen hat,
in einem ersten Schritt mit einem Iodierungsmittel in Gegenwart von Isoamylnitrit umsetzt
und die so erhaltenen 3-Iod-pyrazol-4-carbonsäureester der Formel (XV) 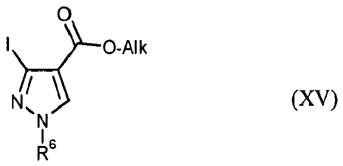 in welcher
Alk die oben angegebenen Bedeutungen hat,
R⁶ die in Anspruch 1 angegebenen Bedeutungen hat,
in einem zweiten Schritt mit einer Base in Gegenwart eines Verdünnungsmittels zur Säure verseift
und diese Säure in einem dritten Schritt gegebenenfalls mit einem Chlorierungsmittel in Gegenwart eines Verdünnungsmittels zum entsprechenden Säurechlorid umsetzt.

## Claims

1. Iodopyrazolylcarboxanilides of the formula (I) 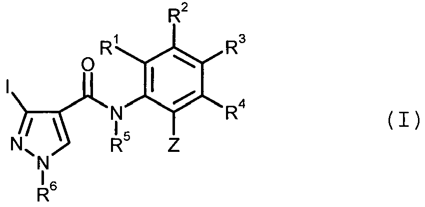 in which
R¹, R², R³ and R⁴ independently of one another represent hydrogen, fluorine, chlorine, methyl, isopropyl or methylthio,
R⁵ represents hydrogen, C₁-C₈-alkyl, C₁-C₆-alkylsulphinyl, C₁-C₆-alkylsulphonyl, C₁-C₄-alkoxy-C₁-C₄-alkyl, C₃-C₈-cycloalkyl; C₁-C₆-haloalkyl, C₁-C₄-haloalkylthio, C₁-C₄-haloalkylsulphinyl, C₁-C₄-haloalkylsulphonyl, halo-C₁-C₄-alkoxy-C₁-C₄-alkyl, C₃-C₈-halocycloalkyl having in each case 1 to 9 fluorine, chlorine and/or bromine atoms, formyl-C₁-C₃-alkyl, (C₁-C₃-alkyl) carbonyl-C₁-C₃-alkyl, (C₁-C₃-alkoxy) - carbonyl-C₁-C₃-alkyl; (C₁-C₃-haloalkyl) - carbonyl-C₁-C₃-alkyl, (C₁-C₃-haloalkoxy) - carbonyl-C₁-C₃-alkyl having in each case 1 to 7 fluorine, chlorine and/or bromine atoms, (C₁-C₃-alkyl) carbonyl-C₁-C₃-haloalkyl, (C₁-C₃-alkoxy)carbonyl-C₁-C₃-haloalkyl having in each case 1 to 6 fluorine, chlorine and/or bromine atoms, (C₁-C₃-haloalkyl) carbonyl-C₁-C₃-haloalkyl, (C₁-C₃-haloalkoxy) carbonyl-C₁-C₃-haloalkyl having in each case 1 to 13 fluorine, chlorine and/or bromine atoms; -COR⁷, -CONR⁸R⁹ or -CH₂NR¹⁰R¹¹,
R⁶ represents C₁-C₃-alkyl, C₁-C₂-alkoxy-C₁-C₂-alkyl, C₁-C₃-haloalkyl having 1 to 7 fluorine, chlorine and/or bromine atoms,
R⁷ represents hydrogen, C₁-C₈-alkyl, C₁-C₈-alkoxy, C₁-C₄-alkoxy-C₁-C₄-alkyl, C₃-C₈-cycloalkyl; C₁-C₆-haloalkyl, C₁-C₆-haloalkoxy, halo-C₁-C₄-alkoxy-C₁-C₄-alkyl, C₃-C₈-halocycloalkyl having in each case 1 to 9 fluorine, chlorine and/or bromine atoms, -COR¹²,
R⁸ and R⁹ independently of one another represent hydrogen, C₁-C₈-alkyl, C₁-C₄-alkoxy-C₁-C₄-alkyl, C₃-C₈-cycloalkyl; C₁-C₈-haloalkyl, halo-C₁-C₄-alkoxy-C₁-C₄-alkyl, C₃-C₈-halocycloalkyl having in each case 1 to 9 fluorine, chlorine and/or bromine atoms,
R⁸ and R⁹ furthermore together with the nitrogen atom to which they are attached form a saturated heterocycle which is optionally mono- or polysubstituted by identical or different substituents from the group consisting of halogen and C₁-C₄-alkyl and which has 5 to 8 ring atoms, where the heterocycle may contain 1 or 2 further nonadjacent heteroatoms from the group consisting of oxygen, sulphur and NR¹³,
R¹⁰ and R¹¹ independently of one another represent hydrogen, C₁-C₈-alkyl, C₃-C₈-cycloalkyl; C₁-C₈-haloalkyl, C₃-C₈-halocycloalkyl having in each case 1 to 9 fluorine, chlorine and/or bromine atoms,
R¹⁰ and R¹¹ furthermore together with the nitrogen atom to which they are attached form a saturated heterocycle which is optionally mono- or polysubstituted by identical or different substituents from the group consisting of halogen and C₁-C₄-alkyl and which has 5 to 8 ring atoms, where the heterocycle may contain 1 or 2 further nonadjacent heteroatoms from the group consisting of oxygen, sulphur and NR¹³,
R¹² represents hydrogen, C₁-C₈-alkyl, C₁-C₈-alkoxy, C₁-C₄-alkoxy-C₁-C₄-alkyl, C₃-C₈-cycloalkyl; C₁-C₆-haloalkyl, C₁-C₆-haloalkoxy, halo-C₁-C₄-alkoxy-C₁-C₄-alkyl, C₃-C₈-halocycloalkyl having in each case 1 to 9 fluorine, chlorine and/or bromine atoms,
R¹³ represents hydrogen or C₁-C₆-alkyl,
Z represents Z¹, where
Z¹ represents phenyl which is optionally mono- to pentasubstituted by identical or different substituents,
or
R¹, R² and R³ independently of one another represent hydrogen, fluorine or chlorine and
Z and R⁴ together with the carbon atoms to which they are attached form an optionally substituted 5- or 6-membered carbocyclic or heterocyclic ring.

2. Iodopyrazolylcarboxanilides of the formula (I) according to Claim 1 in which
R¹, R², R³ and R⁴ independently of one another represent hydrogen, fluorine, chlorine or methyl,
R⁵ represents hydrogen; C₁-C₆-alkyl, C₁-C₄-alkylsulphinyl, C₁-C₄-alkylsulphonyl, C₁-C₃-alkoxy-C₁-C₃-alkyl, C₃-C₆-cycloalkyl; C₁-C₄-haloalkyl, C₁-C₄-haloalkylthio, C₁-C₄-haloalkylsulphinyl, C₁-C₄-haloalkylsulphonyl, halo-C₁-C₃-alkoxy-C₁-C₃-alkyl, C₃-C₆-halocycloalkyl having in each case 1 to 9 fluorine, chlorine and/or bromine atoms, formyl-C₁-C₃-alkyl, (C₁-C₃-alkyl) carbonyl-C₁-C₃-alkyl, (C₁-C₃-alkoxy) - carbonyl-C₁-C₃-alkyl; (C₁-C₃-haloalkyl) - carbonyl-C₁-C₃-alkyl, (C₁-C₃-haloalkoxy) - carbonyl-C₁-C₃-alkyl having in each case 1 to 7 fluorine, chlorine and/or bromine atoms, (C₁-C₃-alkyl) carbonyl-C₁-C₃-haloalkyl, (C₁-C₃-alkoxy)carbonyl-C₁-C₃-haloalkyl having in each case 1 to 6 fluorine, chlorine and/or bromine atoms, (C₁-C₃-haloalkyl) carbonyl-C₁-C₃-haloalkyl, (C₁-C₃-haloalkoxy)carbonyl-C₁-C₃-haloalkyl having in each case 1 to 13 fluorine, chlorine and/or bromine atoms; -COR⁷, -CONR⁸R⁹ or -CH₂NR¹⁰R¹¹,
R⁶ represents methyl, ethyl, isopropyl, monofluoromethyl, difluoromethyl or trifluoromethyl,
R⁷ represents hydrogen, C₁-C₆-alkyl, C₁-C₄-alkoxy, C₁-C₃-alkoxy-C₁-C₃-alkyl, C₃-C₆-cycloalkyl; C₁-C₄-haloalkyl, C₁-C₄-haloalkoxy, halo-C₁-C₃-alkoxy-C₁-C₃-alkyl, C₃-C₆-halocycloalkyl having in each case 1 to 9 fluorine, chlorine and/or bromine atoms, -COR¹²,
R⁸ and R⁹ independently of one another represent hydrogen, C₁-C₆-alkyl, C₁-C₃-alkoxy-C₁-C₃-alkyl, C₃-C₆-cycloalkyl; C₁-C₄-haloalkyl, halo-C₁-C₃-alkoxy-C₁-Cs-alkyl, C₃-C₆-halocycloalkyl having in each case 1 to 9 fluorine, chlorine and/or bromine atoms,
R¹⁰ and R¹¹ independently of one another represent hydrogen, C₁-C₆-alkyl, C₃-C₆-cycloalkyl; C₁-C₄-haloalkyl, C₃-C₆-halocycloalkyl having in each case 1 to 9 fluorine, chlorine and/or bromine atoms,
R¹⁰ and R¹¹ furthermore together with the nitrogen atom to which they are attached form a saturated heterocycle which is optionally mono- or polysubstituted by identical or different substituents from the group consisting of halogen and C₁-C₄-alkyl and which has 5 to 8 ring atoms, where the heterocycle may contain 1 or 2 further nonadjacent heteroatoms from the group consisting of oxygen, sulphur and NR¹³,
R¹² represents hydrogen, C₁-C₆-alkyl, C₁-C₄-alkoxy, C₁-C₃-alkoxy-C₁-C₃-alkyl, C₃-C₆-cycloalkyl; C₁-C₄-haloalkyl, C₁-C₄-haloalkoxy, halo-C₁-C₃-alkoxy-C₁-C₃-alkyl, C₃-C₆-halocycloalkyl having in each case 1 to 9 fluorine, chlorine and/or bromine atoms,
R¹³ represents hydrogen or C₁-C₄-alkyl,
Z represents Z¹, in which
Z¹ represents phenyl which is optionally mono- to pentasubstituted by identical or different substituents, where the substituents are selected from the list W¹,
W¹ represents halogen, cyano, nitro, amino, hydroxyl, formyl, carboxyl, carbamoyl, thiocarbamoyl;
in each case straight-chain or branched alkyl, hydroxyalkyl, oxoalkyl, alkoxy, alkoxyalkyl, alkylthioalkyl, dialkoxyalkyl, alkylthio, alkylsulphinyl or alkylsulphonyl having in each case 1 to 8 carbon atoms;
in each case straight-chain or branched alkenyl or alkenyloxy having in each case 2 to 6 carbon atoms;
in each case straight-chain or branched haloalkyl, haloalkoxy, haloalkylthio, haloalkylsulphinyl or haloalkylsulphonyl having in each case 1 to 6 carbon atoms and 1 to 13 identical or different halogen atoms; in each case straight-chain or branched haloalkenyl or haloalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl, alkylaminocarbonyl, dialkylaminocarbonyl, arylalkylaminocarbonyl, dialkylaminocarbonyloxy having 1 to 6 carbon atoms in the respective hydrocarbon chains, alkenylcarbonyl or alkynylcarbonyl having 2 to 6 carbon atoms in the respective hydrocarbon chains;
cycloalkyl or cycloalkyloxy having in each case 3 to 6 carbon atoms;
in each case doubly attached alkylene having 3 or 4 carbon atoms, oxyalkylene having 2 or 3 carbon atoms or dioxyalkylene having 1 or 2 carbon atoms, each of which radicals is optionally mono- to tetrasubstituted by identical or different substituents from the group consisting of fluorine, chlorine, oxo, methyl, trifluoromethyl or ethyl;
or a grouping -C(Q¹)=N-Q² in which
Q¹ represents hydrogen, hydroxyl, alkyl having 1 to 4 carbon atoms, haloalkyl having 1 to 4 carbon atoms and 1 to 9 identical or different halogen atoms or cycloalkyl having 1 to 6 carbon atoms and
Q² represents hydroxyl, amino, methylamino, phenyl, benzyl or represents in each case optionally halogen-, cyano-, hydroxyl-, alkoxy-, alkylthio-, alkylamino-, dialkylamino- or phenyl-substituted alkyl or alkoxy having 1 to 4 carbon atoms, or represents alkenyloxy or alkynyloxy having in each case 2 to 4 carbon atoms,
and also phenyl, phenoxy, phenylthio, benzoyl, benzoylethenyl, cinnamoyl, heterocyclyl or phenylalkyl, phenylalkyloxy, phenylalkylthio or heterocyclylalkyl having in each case 1 to 3 carbon atoms in the respective alkyl moieties, each of which radicals is optionally mono- to trisubstituted in the cyclic part by halogen and/or straight-chain or branched alkyl or alkoxy having 1 to 4 carbon atoms,
Z and R⁴ together with the carbon atoms to which they are attached also represent a 5- or 6-membered carbocyclic or heterocyclic ring which is optionally mono- to tetrasubstituted by identical or different substituents.

3. Process for preparing the iodopyrazolylcarboxanilides of the formula (I) according to Claim 1, **characterized in that**
a) iodopyrazolylcarboxylic acid derivatives of the formula (II) 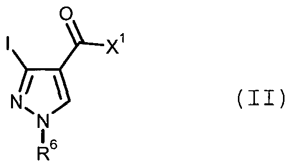 in which
R⁶ is as defined in Claim 1 and
X¹ represents chlorine or hydroxyl
are reacted with aniline derivatives of the formula (III) 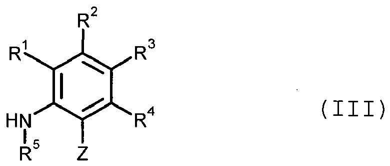 in which R¹, R², R³, R⁴, R⁵ and Z are as defined in Claim 1,
if appropriate in the presence of a catalyst, if appropriate in the presence of a condensing agent, if appropriate in the presence of an acid binder and if appropriate in the presence of a diluent,
or
b) haloiodopyrazolylcarboxanilides of the formula (IV) 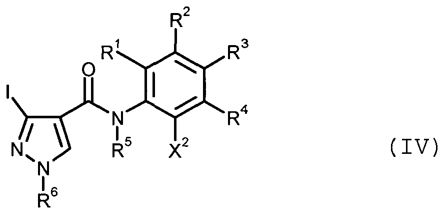 in which
R¹, R², R³, R⁴, R⁵ and R⁶ are as defined in Claim 1 and
X² represents chlorine, bromine, iodine or trifluoromethylsulphonate
are reacted with boronic acid derivatives of the formula (V) 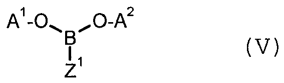 in which
Z¹ is as defined in Claim 1 and
A¹ and A² each represent hydrogen or together represent tetramethylethylene,
in the presence of a catalyst, if appropriate in the presence of an acid binder and if appropriate in the presence of a diluent,
or
c) iodopyrazolylcarboxamide boronic acid derivatives of the formula (VI) 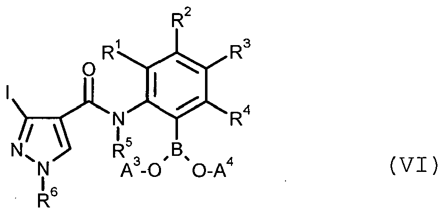 in which
R¹, R², R³, R⁴, R⁵ and R⁶ are as defined in Claim 1 and
A³ and A⁴ each represent hydrogen or together represent tetramethylethylene,
are reacted with phenyl derivatives of the formula (VII)
X—Z¹ (VII)
in which
Z¹ is as defined in Claim 1 and
X³ represents chlorine, bromine, iodine or trifluoromethylsulphonate,
in the presence of a catalyst, if appropriate in the presence of an acid binder and if appropriate in the presence of a diluent,
or
d) haloiodopyrazolylcarboxanilides of the formula (IV) 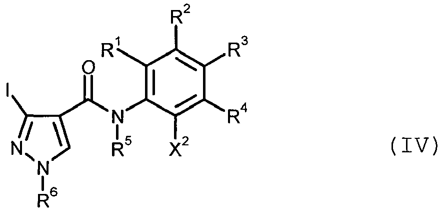 in which
R¹, R², R³, R⁴, R⁵ and R⁶ are as defined in Claim 1, and
X² represents chlorine, bromine, iodine or trifluoromethylsulphonate,
are reacted with phenyl derivatives of the formula (VII)
X³—Z¹ (VII)
in which
Z¹ is as defined in Claim 1 and
X³ represents chlorine, bromine, iodine or trifluoromethylsulphonate,
in the presence of a palladium or nickel catalyst and in the presence of 4, 4, 4' , 4' , 5, 5, 5' , 5' -octamethyl-2, 2' -bis-1, 3, 2-dioxaborolane, if appropriate in the presence of an acid binder and if appropriate in the presence of a diluent,
or
e) iodopyrazolylcarboxanilides of the formula (Ia) 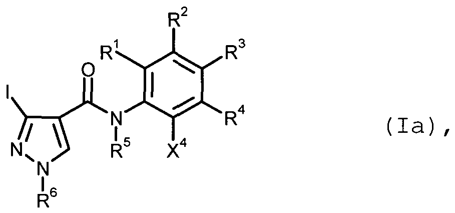 in which
R¹, R², R³, R⁴, R⁵ and R⁶ are as defined in Claim 1 and
X⁴ represents C₂-C₂₀-alkenyl or C₂-C₂₀-alkynyl, each of which is optionally mono- or polysubstituted by identical or different substituents from the group consisting of halogen and C₃-C₆-cycloalkyl, where the cycloalkyl moiety for its part may optionally be substituted by halogen and/or C₁-C₄-alkyl,
are hydrogenated, if appropriate in the presence of a diluent and if appropriate in the presence of a catalyst,
or
f) hydroxyalkyliodopyrazolylcarboxanilides of the formula (VIII) 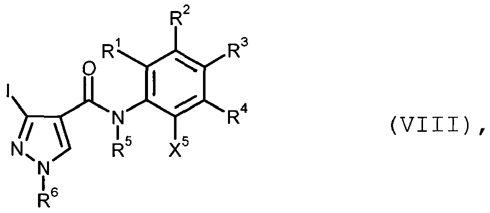 in which
R¹, R², R³, R⁴, R⁵ and R⁶ are as defined in Claim 1 and
X⁵ represents C₂-C₂₀-hydroxyalkyl which is optionally additionally mono- or polysubstituted by identical or different substituents from the group consisting of halogen and C₃-C₆-cycloalkyl, where the cycloalkyl moiety for its part may optionally be substituted by halogen and/or C₁-C₄-alkyl,
are dehydrated, if appropriate in the presence of a diluent and if appropriate in the presence of an acid,
or
g) haloiodopyrazolylcarboxanilides of the formula (IV) 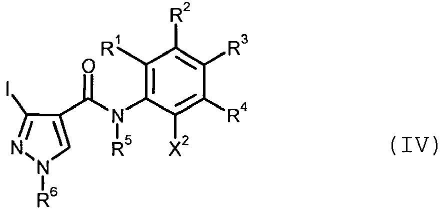 in which
R¹, R², R³, R⁴, R⁵ and R⁶ are as defined in Claim 1, and
X² represents chlorine, bromine, iodine or trifluoromethylsulphonate,
are reacted with an alkyne of the formula (IX) 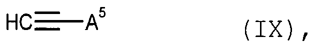 in which
A⁵ represents C₂-C₁₈-alkyl which is optionally mono- or polysubstituted by identical or different substituents from the group consisting of halogen and C₃-C₆-cycloalkyl, where the cycloalkyl moiety for its part may optionally be substituted by halogen and/or C₁-C₄-alkyl,
or an alkene of the formula (X) 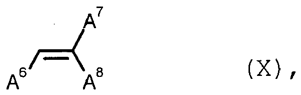 in which
A⁶, A⁷ and A⁸ independently of one another each represent hydrogen or alkyl which is optionally mono- or polysubstituted by identical or different substituents from the group consisting of halogen and C₃-C₆-cycloalkyl, where the cycloalkyl moiety for its part may optionally be substituted by halogen and/or C₁-C₄-alkyl and the total number of carbon atoms of the open-chain part of the molecule does not exceed the number 20,
if appropriate in the presence of a diluent,
if appropriate in the presence of an acid binder and if appropriate in the presence of one or more catalysts,
or
h) ketones of the formula (XI) 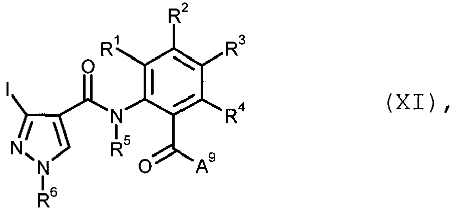 in which
R¹, R², R³, R⁴, R⁵ and R⁶ are as defined in Claim 1 and
A⁹ represents hydrogen or C₁-C₁₈-alkyl which is optionally mono- or polysubstituted by identical or different substituents from the group consisting of halogen and-C₃-C₆-cycloalkyl, where the cycloalkyl moiety for its part may optionally be substituted by halogen and/or C₁-C₄-alkyl,
are reacted with a phosphorus compound of the formula (XII)
A¹⁰—Px (XII),
in which
A¹⁰ represents C₁-C₁₈-alkyl which is optionally mono- or polysubstituted by identical or different substituents from the group consisting of halogen and C₃-C₆-cycloalkyl, where the cycloalkyl moiety for its part may optionally be substituted by halogen and/or C₁-C₄-alkyl,
Px represents a grouping -P⁺(C₆H₅)₃ Cl⁻, - P⁺ (C₆H₅) ₃ Br⁻, -P⁺ (C₆H₅) ₃ I⁻, -P (=O) (OCH₃)₃ or -P (=O) (OC₂H₅)₃,
if appropriate in the presence of a diluent,
or
i) iodopyrazolylcarboxanilides of the formula (Ib) 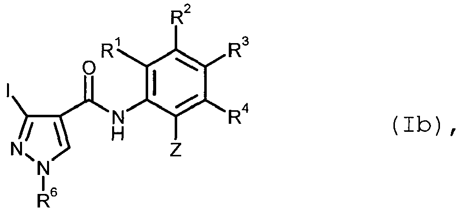 in which
R¹, R², R³, R⁴, R⁶ and Z are as defined in Claim 1,
are reacted with a halide of the formula (XIII)
R⁵⁻¹—X⁶ (XIII)
in which
R⁵⁻¹ represents C₁-C₈-alkyl, C₁-C₆-alkylsulphinyl, C₁-C₆-alkylsulphonyl, C₁-C₄-alkoxy-C₁-C₄-alkyl, C₃-C₈-cycloalkyl; C₁-C₆-haloalkyl, C₁-C₄-haloalkylthio, C₁-C₄-haloalkylsulphinyl, C₁-C₄-haloalkylsulphonyl, halo-C₁-C₄-alkoxy-C₁-C₄-alkyl, C₃-C₈-halocycloalkyl having in each case 1 to 9 fluorine, chlorine and/or bromine atoms, formyl-C₁-C₃-alkyl, (C₁-C₃-alkyl) carbonyl-C₁-C₃-alkyl, (C₁-C₃-alkoxy) carbonyl-C₁-C₃-alkyl; (C₁-C₃-haloalkyl) carbonyl-C₁-C₃-alkyl, (C₁-C₃-haloalkoxy)carbonyl-C₁-C₃-alkyl having in each case 1 to 7 fluorine, chlorine and/or bromine atoms, (C₁-C₃-alkyl) carbonyl-C₁-C₃-haloalkyl, (C₁-C₃-alkoxy)carbonyl-C₁-C₃-haloalkyl having in each case 1 to 6 fluorine, chlorine and/or bromine atoms, (C₁-C₃-haloalkyl) carbonyl-C₁-C₃-haloalkyl, (C₁-C₃-haloalkoxy) carbonyl-C₁-C₃-haloalkyl having in each case 1 to 13 fluorine, chlorine and/or bromine atoms; -COR⁷, -CONR⁸R⁹ or -CH₂NR¹⁰R¹¹,
R⁷, R⁸, R⁹, R¹⁰ and R¹¹ are as defined in Claim 1 and
X⁶ represents chlorine, bromine or iodine,
in the presence of a base and in the presence of a diluent.

4. Composition for controlling unwanted microorganisms, **characterized in that** it comprises at least one iodopyrazolylcarboxanilide of the formula (I) according to Claim 1, in addition to extenders and/or surfactants.

5. Use of iodopyrazolylcarboxanilides of the formula (I) according to Claim 1 for controlling unwanted microorganisms.

6. Method for controlling unwanted microorganisms, **characterized in that** iodopyrazolylcarboxanilides of the formula (I) according to Claim 1 are applied to the microorganisms and/or their habitat.

7. Process for preparing compositions for controlling unwanted microorganisms, **characterized in that** iodopyrazolylcarboxanilides of the formula (I) according to Claim 1 are mixed with extenders and/or surfactants.

8. Hydroxyalkyliodopyrazolylcarboxanilides of the formula (VIII) 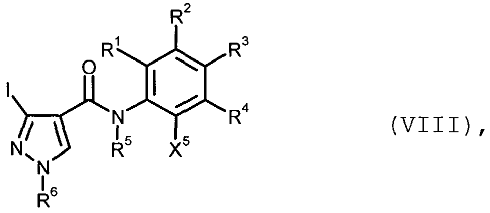 in which
R¹, R², R³, R⁴, R⁵ and R⁶ are as defined in Claim 1,
X⁵ represents C₂-C₂₀-hydroxyalkyl which is optionally additionally mono- or polysubstituted by identical or different substituents from the group consisting of halogen and C₃-C₆-cycloalkyl, where the cycloalkyl moiety for its part may optionally be substituted by halogen and/or C₁-C₄-alkyl.

9. Iodopyrazolylcarboxylic acid derivatives of the formula (II) 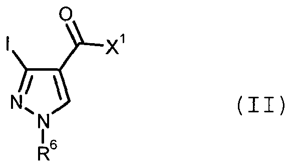 in which
R⁶ is as defined in Claim 1 and
X¹ represents chlorine or hydroxyl.

10. Process for preparing iodopyrazolylcarboxylic acid derivatives of the formula (II) according to Claim 9, **characterized in that**
j) 3-aminopyrazole-4-carboxylic esters of the formula (XIV) 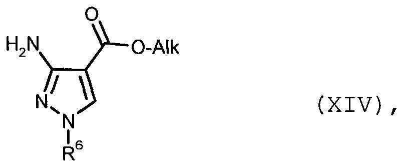 in which
Alk represents C₁-C₄-alkyl and
R⁶ is as defined in Claim 1,
are reacted in a first step with an iodonating agent in the presence of isoamyl nitrite and the resulting 3-iodopyrazole-4-carboxylic esters of the formula (XV) 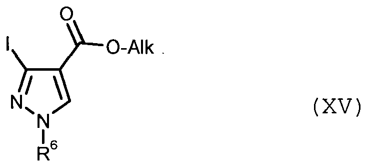 in which
Alk is as defined above,
R⁶ is as defined in Claim 1,
are in a second step hydrolyzed to the acid using a base in the presence of a diluent
and this acid is, if appropriate, reacted in a third step with a chlorinating agent in the presence of a diluent to give the corresponding acid chloride.

## Revendications

1. Iodopyrazolylcarboxanilides de formule (I) 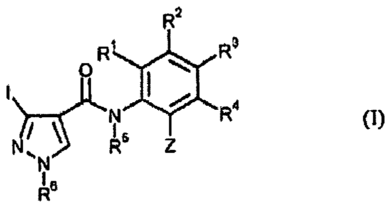 dans laquelle
R¹, R², R³ et R⁴ représentent indépendamment les uns des autres hydrogène, fluor, chlore, méthyle, isopropyle ou méthylthio,
R⁵ représente hydrogène, alkyle en C₁-C₈, alkylsulfinyle en C₁-C₆, alkylsulfonyle en C₁-C₆, alcoxy en C₁-C₄-alkyle en C₁-C₄, cycloalkyle en C₃-C₈ ; halogénoalkyle en C₁-C₆, halogénoalkylthio en C₁-C₄, halogénoalkylsulfinyle en C₁-C₄, halogénoalkylsulfonyle en C₁-C₄, halogéno-alcoxy en C₁-C₄-alkyle en C₁-C₄, halogénocycloalkyle en C₃-C₈ contenant à chaque fois 1 à 9 atomes de fluor, chlore et/ou brome, formyl-alkyle en C₁-C₃, (alkyle en C₁-C₃) carbonyl-alkyle en C₁-C₃, (alcoxy en C₁-C₃ carbonyl-alkyle en C₁-C₃; (halogénoalkyle en C₁-C₃) carbonyl-alkyle en C₁-C₃, (halogénoalcoxy en C₁-C₃,)carbonyl-alkyle en C₁-C₃ contenant à chaque fois 1 à 7 atomes de fluor, chlore et/ou brome, (alkyle en C₁-C₃) carbonyl-halogénoalkyle en C₁-C₃, (alcoxy en C₁-C₃)carbonyl-halogénoalkyle en C₁-C₃ contenant à chaque fois 1 à 6 atomes de fluor, chlore et/ou brome, (halogénoalkyle en C₁-C₃) carbonyl-halogénoalkyle en C₁-C₃, (halogénoalcoxy en C₁-C₃) carbonyl-halogénoalkyle en C₁-C₃ contenant à chaque fois 1 à 13 atomes de fluor, chlore et/ou brome ; -COR⁷, -CONR⁸R⁹ ou -CH₂NR¹⁰R¹¹,
R⁶ représente alkyle en C₁-C₃, alcoxy en C₁-C₂-alkyle en C₁-C₂, halogénoalkyle en C₁-C₃ contenant 1 à 7 atomes de fluor, chlore et/ou brome,
R⁷ représente hydrogène, alkyle en C₁-C₈, alcoxy en C₁-C₈, alcoxy en C₁-C₄-alkyle en C₁-C₄, cycloalkyle en C₃-C₈ ; halogénoalkyle en C₁-C₆, halogénoalcoxy en C₁-C₆, halogéno-alcoxy en C₁-C₄-alkyle en C₁-C₄, halogénocycloalkyle en C₃-C₈ contenant à chaque fois 1 à 9 atomes de fluor, chlore et/ou brome, -COR¹²,
R⁸ et R⁹ représentent indépendamment l'un de l'autre hydrogène, alkyle en C₁-C₈, alcoxy en C₁-C₄-alkyle en C₁-C₄, cycloalkyle en C₃-C₈ ; halogénoalkyle en C₁-C₈, halogéno-alcoxy en C₁-C₄-alkyle en C₁-C₄, halogénocycloalkyle en C₃-C₈ contenant à chaque fois 1 à 9 atomes de fluor, chlore et/ou brome,
R⁸ et R⁹ forment également ensemble avec l'atome d'azote auquel ils sont reliés un hétérocycle saturé de 5 à 8 atomes de cycle, éventuellement substitué une ou plusieurs fois, de manière identique ou différente, par halogène ou alkyle en C₁-C₄, l'hétérocycle pouvant contenir 1 ou 2 hétéroatomes non voisins supplémentaires de la série constituée par oxygène, soufre ou NR¹³,
R¹⁰ et R¹¹ représentent indépendamment l'un de l'autre hydrogène, alkyle en C₁-C₈, cycloalkyle en C₃-C₈ ; halogénoalkyle en C₁-C₈, halogénocycloalkyle en C₃-C₈ contenant à chaque fois 1 à 9 atomes de fluor, chlore et/ou brome,
R¹⁰ et R¹¹ forment également ensemble avec l'atome d'azote auquel ils sont reliés un hétérocycle saturé de 5 à 8 atomes de cycle, éventuellement substitué une ou plusieurs fois, de manière identique ou différente, par halogène ou alkyle en C₁-C₄, l'hétérocycle pouvant contenir 1 ou 2 hétéroatomes non voisins supplémentaires de la série constituée par oxygène, soufre ou NR¹³,
R¹² représente hydrogène, alkyle en C₁-C₈, alcoxy en C₁-C₈, alcoxy en C₁-C₄-alkyle en C₁-C₄, cycloalkyle en C₃-C₈ ; halogénoalkyle en C₁-C₆, halogénoalcoxy en C₁-C₆, halogéno-alcoxy en C₁-C₄-alkyle en C₁-C₄, halogénocycloalkyle en C₃-C₈ contenant à chaque fois 1 à 9 atomes de fluor, chlore et/ou brome,
R¹³ représente hydrogène ou alkyle en C₁-C₆,
Z représente Z¹,
Z¹ représentant phényle éventuellement substitué une à cinq fois, de manière identique ou différente,
ou
R¹, R² et R³ représentent indépendamment les uns des autres hydrogène, fluor ou chlore, et
Z et R⁴ forment ensemble avec les atomes de carbone auxquels ils sont reliés un cycle carbocyclique ou hétérocyclique à 5 ou 6 éléments, éventuellement substitué.

2. Iodopyrazolylcarboxanilides de formule (I) selon la revendication 1, dans laquelle
R¹, R², R³ et R⁴ représentent indépendamment les uns des autres hydrogène, fluor, chlore ou méthyle,
R⁵ représente hydrogène ; alkyle en C₁-C₆, alkylsulfinyle en C₁-C₄, alkylsulfonyle en C₁-C₄, alcoxy en C₁-C₃-alkyle en C₁-C₃, cycloalkyle en C₃-C₆ ; halogénoalkyle en C₁-C₄, halogénoalkylthio en C₁-C₄, halogénoalkylsulfinyle en C₁-C₄, halogénoalkylsulfonyle en C₁-C₄, halogéno-alcoxy en C₁-C₃-alkyle en C₁-C₃, halogénocycloalkyle en C₃-C₆ contenant à chaque fois 1 à 9 atomes de fluor, chlore et/ou brome, formyl-alkyle en C₁-C₃, (alkyle en C₁-C₃) carbonyl-alkyle en C₁-C₃, (alcoxy en C₁-C₃) carbonyl-alkyle en C₁-C₃; (halogénoalkyle en C₁-C₃) carbonyl-alkyle en C₁-C₃, (halogénoalcoxy en C₁-C₃,)carbonyl-alkyle en C₁-C₃ contenant à chaque fois 1 à 7 atomes de fluor, chlore et/ou brome, (alkyle en C₁-C₃) carbonyl-halogénoalkyle en C₁-C₃, (alcoxy en C₁-C₃)carbonyl-halogénoalkyle en C₁-C₃ contenant à chaque fois 1 à 6 atomes de fluor, chlore et/ou brome, (halogénoalkyle en C₁-C₃)carbonyl-halogénoalkyle en C₁-C₃, (halogénoalcoxy en C₁-C₃) carbonyl-halogénoalkyle en C₁-C₃ contenant à chaque fois 1 à 13 atomes de fluor, chlore et/ou brome ; -COR⁷, -CONR⁸R⁹ ou -CH₂NR¹⁰R¹¹,
R⁶ représente méthyle, éthyle, isopropyle, monofluorométhyle, difluorométhyle ou trifluorométhyle,
R⁷ représente hydrogène, alkyle en C₁-C₆, alcoxy en C₁-C₄, alcoxy en C₁-C₃-alkyle en C₁-C₃, cycloalkyle en C₃-C₆ ; halogénoalkyle en C₁-C₄, halogénoalcoxy en C₁-C₄, halogéno-alcoxy en C₁-C₃-alkyle en C₁-C₃, halogénocycloalkyle en C₃-C₆ contenant à chaque fois 1 à 9 atomes de fluor, chlore et/ou brome, -COR¹²,
R⁸ et R⁹ représentent indépendamment l'un de l'autre hydrogène, alkyle en C₁-C₆, alcoxy en C₁-C₃-alkyle en C₁-C₃, cycloalkyle en C₃-C₆ ; halogénoalkyle en C₁-C₄, halogéno-alcoxy en C₁-C₃-alkyle en C₁-C₃, halogénocycloalkyle en C₃-C₆ contenant à chaque fois 1 à 9 atomes de fluor, chlore et/ou brome,
R¹⁰ et R¹¹ représentent indépendamment l'un de l'autre hydrogène, alkyle en C₁-C₆, cycloalkyle en C₃-C₆; halogénoalkyle en C₁-C₄, halogénocycloalkyle en C₃-C₆ contenant à chaque fois 1 à 9 atomes de fluor, chlore et/ou brome,
R¹⁰ et R¹¹ forment également ensemble avec l'atome d'azote auquel ils sont reliés un hétérocycle saturé de 5 à 8 atomes de cycle, éventuellement substitué une ou plusieurs fois, de manière identique ou différente, par halogène ou alkyle en C₁-C₄, l'hétérocycle pouvant contenir 1 ou 2 hétéroatomes non voisins supplémentaires de la série constituée par oxygène, soufre ou NR¹³,
R¹² représente hydrogène, alkyle en C₁-C₆, alcoxy en C₁-C₄, alcoxy en C₁-C₃-alkyle en C₁-C₃, cycloalkyle en C₃-C₆ ; halogénoalkyle en C₁-C₄, halogénoalcoxy en C₁-C₄, halogéno-alcoxy en C₁-C₃-alkyle en C₁-C₃, halogénocycloalkyle en C₃-C₆ contenant à chaque fois 1 à 9 atomes de fluor, chlore et/ou brome,
R¹³ représente hydrogène ou alkyle en C₁-C₄,
Z représente Z¹,
Z¹ représentant phényle éventuellement substitué une à cinq fois, de manière identique ou différente, les substituants étant choisis dans la liste W¹,
W¹ représente halogène, cyano, nitro, amino, hydroxy, formyle, carboxyle, carbamoyle, thiocarbamoyle ;
alkyle, hydroxyalkyle, oxoalkyle, alcoxy, alcoxyalkyle, alkylthioalkyle, dialcoxyalkyle, alkylthio, alkylsulfinyle ou alkylsulfonyle à chaque fois linéaire ou ramifié, contenant à chaque fois 1 à 8 atomes de carbone ;
alcényle ou alcényloxy à chaque fois linéaire ou ramifié, contenant à chaque fois 2 à 6 atomes de carbone ;
halogénoalkyle, halogénoalcoxy, halogénoalkylthio, halogénoalkylsulfinyle ou halogénoalkylsulfonyle à chaque fois linéaire ou ramifié, contenant à chaque fois 1 à 6 atomes de carbone et 1 à 13 atomes d'halogène identiques ou différents ;
halogénoalcényle ou halogénoalcényloxy à chaque fois linéaire ou ramifié, contenant à chaque fois 2 à 6 atomes de carbone et 1 à 11 atomes d'halogène identiques ou différents ;
alkylamino, dialkylamino, alkylcarbonyle, alkylcarbonyloxy, alcoxycarbonyle, alkylaminocarbonyle, dialkylaminocarbonyle, arylalkylaminocarbonyle, dialkylaminocarbonyloxy contenant 1 à 6 atomes de carbone dans les chaînes hydrocarbonées respectives, alcénylcarbonyle ou alcynylcarbonyle contenant 2 à 6 atomes de carbone dans les chaînes hydrocarbonées respectives, à chaque fois linéaire ou ramifié ;
cycloalkyle ou cycloalkyloxy contenant à chaque fois 3 à 6 atomes de carbone ;
alkylène contenant 3 ou 4 atomes de carbone, oxyalkylène contenant 2 ou 3 atomes de carbone ou dioxyalkylène contenant 1 ou 2 atomes de carbone, à chaque fois relié deux fois, à chaque fois éventuellement substitué une à quatre fois, de manière identique ou différente, par fluor, chlore, oxo, méthyle, trifluorométhyle ou éthyle ;
ou un groupe -C (Q¹) =N-Q²,
Q¹ représentant hydrogène, hydroxy, alkyle contenant 1 à 4 atomes de carbone, halogénoalkyle contenant 1 à 4 atomes de carbone et 1 à 9 atomes d'halogène identiques ou différents, ou cycloalkyle contenant 1 à 6 atomes de carbone, et
Q² représentant hydroxy, amino, méthylamino, phényle, benzyle, ou alkyle ou alcoxy contenant 1 à 4 atomes de carbone, à chaque fois éventuellement substitué par halogène, cyano, hydroxy, alcoxy, alkylthio, alkylamino, dialkylamino ou phényle, ou alcényloxy ou alcynyloxy contenant à chaque fois 2 à 4 atomes de carbone,
ainsi que phényle, phénoxy, phénylthio, benzoyle, benzoyléthényle, cinnamoyle, hétérocyclyle ou phénylalkyle, phénylalkyloxy, phénylalkylthio ou hétérocyclylalkyle, contenant à chaque fois 1 à 3 atomes de carbone dans les parties alkyle respectives, à chaque fois éventuellement substitué dans la partie cyclique une à trois fois par halogène et/ou alkyle ou alcoxy linéaire ou ramifié contenant 1 à 4 atomes de carbone,
Z et R⁴ forment également ensemble avec les atomes de carbone auxquels ils sont reliés un cycle carbocyclique ou hétérocyclique à 5 ou 6 éléments, éventuellement substitué une à quatre fois, de manière identique ou différente.

3. Procédé de fabrication des iodopyrazolylcarboxanilides de formule (I) selon la revendication 1, **caractérisé en ce que**
a) des dérivés de l'acide iodopyrazolylcarboxylique de formule (II) 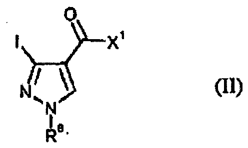 dans laquelle
R⁶ a les significations indiquées dans la revendication 1 et
X¹ représente chlore ou hydroxy,
sont mis en réaction avec des dérivés d'aniline de formule (III) 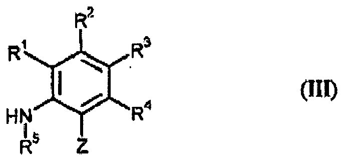 dans laquelle R¹, R², R³, R⁴, R⁵ et Z ont les significations indiquées dans la revendication 1, éventuellement en présence d'un catalyseur, éventuellement en présence d'un agent de condensation, éventuellement en présence d'un liant d'acide et éventuellement en présence d'un diluant,
ou
b) des halogéno-iodopyrazolylcarboxanilides de formule (IV) 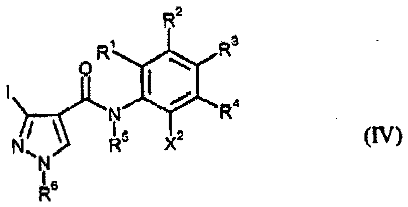 dans laquelle
R¹, R², R³, R⁴, R⁵ et R⁶ ont les significations indiquées dans la revendication 1, et
X² représente chlore, brome, iode ou sulfonate de trifluorométhyle,
sont mis en réaction avec des dérivés de l'acide borique de formule (V) 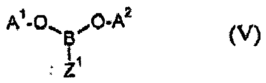 dans laquelle
Z¹ a la signification indiquée dans la revendication 1 et
A¹ et A² représentent chacun hydrogène ou forment ensemble tétraméthyléthylène,
en présence d'un catalyseur, éventuellement en présence d'un liant d'acide et éventuellement en présence d'un diluant,
ou
c) des dérivés d'iodopyrazolylcarboxamide de l'acide borique de formule (VI) 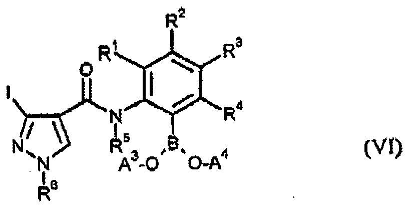 dans laquelle
R¹, R², R³, R⁴, R⁵ et R⁶ ont les significations indiquées dans la revendication 1, et
A³ et A⁴ représentent chacun hydrogène ou forment ensemble tétraméthyléthylène,
sont mis en réaction avec des dérivés de phényle de formule (VII)
X³-Z¹ (VII)
dans laquelle
Z¹ a les significations indiquées dans la revendication 1 et
X³ représente chlore, brome, iode ou sulfonate de trifluorométhyle,
en présence d'un catalyseur, éventuellement en présence d'un liant d'acide et éventuellement en présence d'un diluant,
ou
d) des halogéno-iodopyrazolylcarboxanilides de formule (IV) 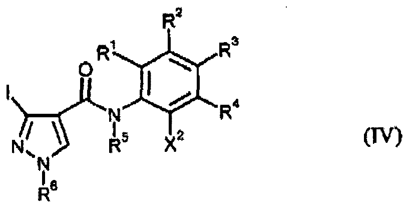 dans laquelle
R¹, R², R³, R⁴, R⁵ et R⁶ ont les significations indiquées dans la revendication 1, et
X² représente chlore, brome, iode ou sulfonate de trifluorométhyle,
sont mis en réaction avec des dérivés de phényle de formule (VII)
X³-Z¹ (VII)
dans laquelle
Z¹ a les significations indiquées dans la revendication 1, et
X³ représente chlore, brome, iode ou sulfonate de trifluorométhyle,
en présence d'un catalyseur de palladium ou de nickel et en présence de 4,4,4',4',5,5,5',5'-octaméthyl-2,2'-bis-1,3,2-dioxaborolane, éventuellement en présence d'un liant d'acide et éventuellement en présence d'un diluant
ou
e) des iodopyrazolylcarboxanilides de formule (Ia) 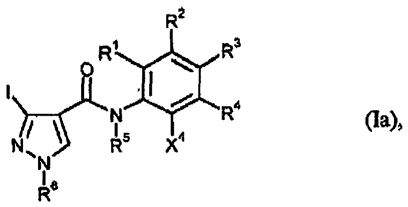 dans laquelle
R¹, R², R³, R⁴, R⁵ et R⁶ ont les significations indiquées dans la revendication 1, et
X⁴ représente alcényle en C₂-C₂₀ ou alcynyle en C₂-C₂₀, à chaque fois éventuellement substitué une ou plusieurs fois, de manière identique ou différente, par halogène et/ou cycloalkyle en C₃-C₆, la partie cycloalkyle pouvant de son côté éventuellement être substituée par halogène et/ou alkyle en C₁-C₄,
sont hydrogénés éventuellement en présence d'un diluant et éventuellement en présence d'un catalyseur,
ou
f) des hydroxyalkyl-iodopyrazolylcarboxanilides de formule (VIII) 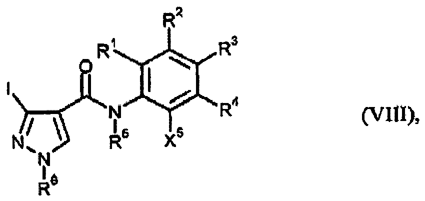 dans laquelle
R¹, R², R³, R⁴, R⁵ et R⁶ ont les significations indiquées dans la revendication 1 et
X⁵ représente hydroxyalkyle en C₂-C₂₀ éventuellement également substitué une ou plusieurs fois, de manière identique ou différente, par halogène et/ou cycloalkyle en C₃-C₆, la partie cycloalkyle pouvant de son côté éventuellement être substituée par halogène et/ou alkyle en C₁-C₄,
sont déshydratés éventuellement en présence d'un diluant et éventuellement en présence d'un acide,
ou
g) des halogéno-iodopyrazolylcarboxanilides de formule (IV) 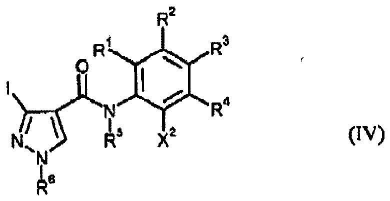 dans laquelle
R¹, R², R³, R⁴, R⁵ et R⁶ ont les significations indiquées dans la revendication 1 et
X² représente chlore, brome, iode ou sulfonate de trifluorométhyle,
sont mis en réaction avec un alcyne de formule (IX) 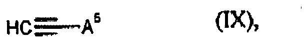 dans laquelle
A⁵ représente alkyle en C₂-C₁₈ éventuellement substitué une ou plusieurs fois, de manière identique ou différente, par halogène et/ou cycloalkyle en C₃-C₆, la partie cycloalkyle pouvant de son côté éventuellement être substituée par halogène et/ou alkyle en C₁-C₄,
ou un alcène de formule (X) 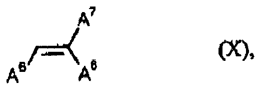 dans laquelle
A⁶, A⁷ et A⁸ représentent chacun indépendamment les uns des autres hydrogène ou alkyle éventuellement substitué une ou plusieurs fois, de manière identique ou différente, par halogène et/ou cycloalkyle en C₃-C₆, la partie cycloalkyle pouvant de son côté éventuellement être substituée par halogène et/ou alkyle en C₁-C₄ et le nombre total d'atomes de carbone de la partie à chaîne ouverte de la molécule ne dépassant pas le nombre 20,
éventuellement en présence d'un diluant, éventuellement en présence d'un liant d'acide et en présence d'un ou de plusieurs catalyseurs,
ou
h) des cétones de formule (XI) 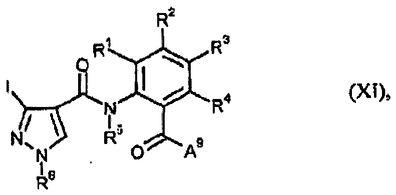 dans laquelle
R¹, R², R³, R⁴, R⁵ et R⁶ ont les significations indiquées dans la revendication 1 et
A⁹ représente hydrogène ou alkyle en C₁-C₁₈ éventuellement substitué une ou plusieurs fois, de manière identique ou différente, par halogène et/ou cycloalkyle en C₃-C₆, la partie cycloalkyle pouvant de son côté éventuellement être substituée par halogène et/ou alkyle en C₁-C₄,
sont mises en réaction avec un composé de phosphore de formule générale (XII)
A¹⁰-Px (XII),
dans laquelle
A¹⁰ représente alkyle en C₁-C₁₈ éventuellement substitué une ou plusieurs fois, de manière identique ou différente, par halogène et/ou cycloalkyle en C₃-C₆, la partie cycloalkyle pouvant de son côté éventuellement être substituée par halogène et/ou alkyle en C₁-C₄,
Px représente un groupe -P⁺(C₆H₅)₃Cl⁻, -P⁺(C₆H₅)₃Br⁻, - P+ (C₆H₅)₃I⁻, -P (=O) (OCH₃) ₃ ou -P(=O) (OC₂H₅)_{3,}
éventuellement en présence d'un diluant,
ou
i) des iodopyrazolylcarboxanilides de formule (Ib) 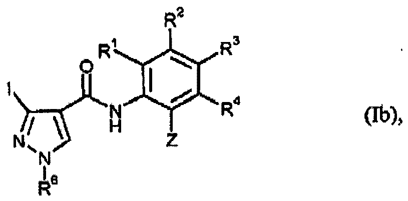
dans laquelle
R¹, R², R³, R⁴, R⁶ et Z ont les significations indiquées dans la revendication 1,
sont mis en réaction avec un halogénure de formule (XIII)
R⁵⁻¹-X⁶ (XIII)
dans laquelle
R⁵⁻¹ représente alkyle en C₁-C₈, alkylsulfinyle en C₁-C₆, alkylsulfonyle en C₁-C₆, alcoxy en C₁-C₄-alkyle en C₁-C₄, cycloalkyle en C₃-C₈ ; halogénoalkyle en C₁-C₆, halogénoalkylthio en C₁-C₄, halogénoalkylsulfinyle en C₁-C₄, halogénoalkylsulfonyle en C₁-C₄, halogéno-alcoxy en C₁-C₄-alkyle en C₁-C₄, halogénocycloalkyle en C₃-C₈ contenant à chaque fois 1 à 9 atomes de fluor, chlore et/ou brome, formyl-alkyle en C₁-C₃, (alkyle en C₁-C₃) carbonyl-alkyle en C₁-C₃, (alcoxy en C₁-C₃) carbonyl-alkyle en C₁-C₃ ; (halogénoalkyle en C₁-C₃) carbonyl-alkyle en C₁-C₃, (halogénoalcoxy en C₁-C₃) carbonyl-alkyle en C₁-C₃ contenant à chaque fois 1 à 7 atomes de fluor, chlore et/ou brome, (alkyle en C₁-C₃)carbonyl-halogénoalkyle en C₁-C₃, (alcoxy en C₁-C₃) carbonyl-halogénoalkyle en C₁-C₃ contenant à chaque fois 1 à 6 atomes de fluor, chlore et/ou brome, (halogénoalkyle en C₁-C₃) carbonyl-halogénoalkyle en C₁-C₃, (halogénoalcoxy en C₁-Cₛ) carbonyl-halogénoalkyle en C₁-C₃ contenant à chaque fois 1 à 13 atomes de fluor, chlore et/ou brome ; -COR⁷, -CONR⁸R⁹ ou -CH₂NR¹⁰R¹¹,
R⁷, R⁸, R⁹, R¹⁰ et R¹¹ ont les significations indiquées dans la revendication 1, et
X⁶ représente chlore, brome ou iode,
en présence d'une base et en présence d'un diluant.

4. Agent pour lutter contre les microorganismes indésirables, **caractérisé par** une teneur en au moins un iodopyrazolylcarboxanilide de formule (I) selon la revendication 1 en plus d'extendeurs et/ou de tensioactifs.

5. Utilisation d'iodopyrazolylcarboxanilides de formule (I) selon la revendication 1 pour lutter contre les microorganismes indésirables.

6. Procédé de lutte contre les microorganismes indésirables, **caractérisé en ce que** des iodopyrazolylcarboxanilides de formule (I) selon la revendication 1 sont appliqués sur les microorganismes et/ou leur habitat.

7. Procédé de fabrication d'agents pour lutter contre les microorganismes indésirables, **caractérisé en ce que** des iodopyrazolylcarboxanilides de formule (I) selon la revendication 1 sont mélangés avec des extendeurs et/ou des tensioactifs.

8. Hydroxyalkyl-iodopyrazolylcarboxanilides de formule (VIII) 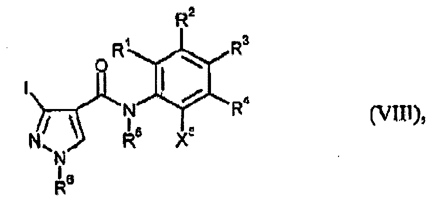 dans laquelle
R¹, R², R³, R⁴, R⁵ et R⁶ ont les significations indiquées dans la revendication 1,
X⁵ représente hydroxyalkyle en C₂-C₂₀ éventuellement également substitué une ou plusieurs fois, de manière identique ou différente, par halogène et/ou cycloalkyle en C₃-C₆, la partie cycloalkyle pouvant de son côté éventuellement être substituée par halogène et/ou alkyle en C₁-C₄.

9. Dérivés de l'acide iodopyrazolylcarboxylique de formule (II) 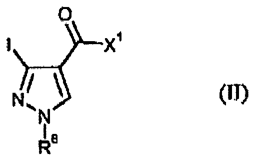 dans laquelle
R⁶ a les significations indiquées dans la revendication 1 et
X¹ représente chlore ou hydroxy.

10. Procédé de fabrication de dérivés de l'acide iodopyrazolylcarboxylique de formule (II) selon la revendication 9, **caractérisé en ce que**
j) des esters de l'acide 3-amino-pyrazol-4-carboxylique de formule (XIV) 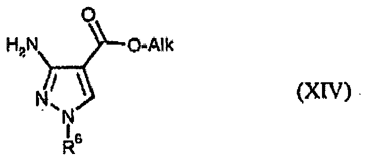 dans laquelle
Alk représente alkyle en C₁-C₄ et
R⁶ a les signification indiquées dans la revendication 1,
sont mis en réaction lors d'une première étape avec un agent d'iodation en présence de nitrite d'isoamyle,
et les esters de l'acide 3-iodo-pyrazol-4-carboxylique de formule (XV) ainsi obtenus 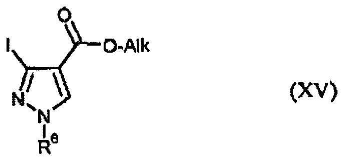 dans laquelle
Alk a les significations indiquées précédemment,
R⁶ a les significations indiquées dans la revendication 1,
est saponifié en l'acide lors d'une deuxième étape avec une base en présence d'un diluant,
et cet acide est transformé en le chlorure d'acide correspondant lors d'une troisième étape, éventuellement avec un agent de chloration, en présence d'un diluant.
